# EUROPEAN PATENT APPLICATION

(11) **EP 4 733 399 A1**
(43) Date of publication of application: **29.04.2026**
(21) Application number: 23943076.2
(22) Date of filing: 07.07.2023
(51) Int. Cl.: C12N 15/00, C12N 15/63, A61K 48/00, A61P 35/00

(54) **METHOD FOR CELL REJUVENATION THROUGH INITIATION-PHASE REPROGRAMMING MEDIATED BY NON-PHASE-SEPARATING TRANSCRIPTION FACTORS**

(30) Priority: 30.06.2023 CN 202310793813
(71) Applicant: Shenzhen Genturn Life Co., Ltd., Shenzhen, Guangdong 518000 (CN)
(72) Inventor: ZHU, Detu, Shenzhen, Guangdong 518000 (CN); ZHOU, Xiangjun, Shenzhen, Guangdong 518000 (CN)
(74) Representative: ABG Intellectual Property Law, S.L.
(86) International application number: PCT/CN2023/106320
(87) International publication number: WO 2025/000588

(57) **Abstract**

Provided in the present invention is a method for cell rejuvenation through initiation-phase reprogramming mediated by phase separation-deficient transcription factors. By modifying transcription factors to not have phase-separation capacity, constructing a recombinant vector and introducing same into a target cell, and controlling the cell to be in the initiation phase of reprogramming, the technical effect of rejuvenation is achieved.

## Description

### Technical field

The present invention relates to the fields of genetic engineering, gene therapy, and cell therapy.

### Background

An important approach for induced pluripotent stem cell (iPSC) reprogramming technology is using transcription factors such as OCT4, SOX2, KLF4, and MYC (hereinafter referred to as 4-factor) to induce differentiated cells into pluripotent stem cells, the process of which is called complete reprogramming, and can be divided into initiation phase, maturation phase, and stabilization phase (Koji et al., 2013; Nelly et al., 2019). During the reprogramming process, cells undergo drastic changes in transcription levels and epigenetic modifications, resulting in dramatic changes in cell identity and state. During the initiation phase, the expression levels of early-stage pluripotency related genes such as TRA-1-60, NANOG, SSEA4, SALL4, etc. rapidly increase, while the expression levels of somatic identity genes do not change much. During the maturation phase, in addition to the early-stage pluripotency related genes mentioned above, maturation specific pluripotency related genes such as DPPA5 and LIN28 are also highly expressed, while somatic identity genes with little change in expression levels during the initiation phase rapidly decrease (Koji et al., 2013, Nelly et al., 2019), suggesting that cells have difficulty in maintaining their original somatic identity at this phase. During the stabilization phase, cells have completely established a pluripotency gene expression network and become iPSCs, which express the above pluripotency related genes and also classical core pluripotency transcription factors such as OCT4, SOX2, KLF4, at high levels, while somatic identity genes are basically not expressed. In addition, this process can also lead to cell rejuvenation, which can be detected by changes in the transcription levels of specific gene, cellular senescence markers, especially epigenetic age (epiAge) based on DNA methylation (Nelly et al., 2019, Antoine et al., 2022, Dilje et al., 2022).

Partial reprogramming refers to the cessation of complete reprogramming in the middle of the process mentioned above. Since cell rejuvenation occurs in the early-stage of reprogramming, and the loss of somatic identity occurs in the middle- and late-stages of reprogramming process, there is a time window to rejuvenate cells while maintaining their original identity, which is the technical principle of partial reprogramming to rejuvenate cells (Ocampo et al. 2016). The main methods of reversing age using transcription factors are currently reported as follows: 1. *In vitro* induction of time-restricted partial reprogramming, also known as transient reprogramming. For example, limiting the overexpression time of 4-factor by an inducible expression system to restrict the reprogramming of human fibroblasts in the initiation phase, which can reverse cell age and maintain the fibroblast identity (Nelly et al., 2019, Dilje et al., 2022); 2. Reprogramming of transient pulses *in vivo,* for example, transient pulse induction of 4-factor overexpression with an inducible system in mice, can make the organs of progeria mice younger and also make each organs of old wild-type mice younger to a certain extent (Alejandro et al., 2016, Kristen et al., 2022); 3. Reducing the sustained induction of partial reprogramming by transcription factors (hereinafter referred to as 3-factor reprogramming), for example, delivering AAV viruses loaded with OSK transcription factor sequences into mouse eyeballs, can treat glaucoma (Lu et al. 2020). The above all are studies that have utilized overexpression of transcription factors to reverse aging, but there is still great room for improvement in practical applications that avoid inducing pluripotent stem cell generation and effectively reverse aging. For example, it is impossible to restrict the expression time of 4-factor by small molecule inducible expression systems in the human body. Therefore, it is necessary to limit the risk of iPSC generation at the source through means such as engineering transcription factors and so on, without affecting the effect of cell rejuvenation.

Phase separation refers to a phenomenon in which some proteins or nucleic acid molecules within a cell can interact with each other through multivalent interactions, to produce another phase with different physical and chemical properties in the surrounding originally homogeneous environment, usually showing droplet-like characteristics, and the result is the formation of membrane-free organelles or cellular structures. This phenomenon or structure is known as liquid-liquid phase separation (LLPS), abbreviated as phase separation (Clifford, et al. 2009, Li, et al. 2012). Phase separation describes specific structures in size at the micron level, creating a relatively independent spatial region that selectively enriches molecules; cell structures formed by phase separation typically have higher protein densities and reduced molecular micromotility compared to the surrounding environment, which can facilitate certain biochemical reactions. The structural characteristics of proteins that can form phase separation are still under study.

The present invention utilizes engineered transcription factors with phase separation capability removed (hereinafter referred to as phase separation-deficient transcription factors) to participate in multi-factor partial reprogramming. The inventor discovered that phase separation-deficient transcription factors causes partial reprogramming to stagnate in the initiation phase, resulting in cell rejuvenation while maintaining cell identity. Therefore, the inventor referred to this as initiation phase reprogramming technique. This technology greatly reduces the risk of iPSC generation while improving the efficiency of cell rejuvenation, making it more promising for clinical applications.

### Summary of the invention

### Definition of General Terms

In the claims and/or specification, when used in conjunction with the term "comprising", the word "a" or "an" may refer to "one", but may also refer to "one or more", "at least one", and "one or more than one".

In the present disclosure, "comprising", "including", "containing", "having" and the like have the meanings that are assigned by the Patent Laws of China, the United States, Europe and other countries, and may refer to "embracing", "covering" and the like. "Essentially consisting of', "essentially composed of" and the like have the meanings that are assigned by the patent laws of China, the United States, Europe and other countries, which are open-ended and allow for the existence of more than the cited matters, as long as the basic or novel features of the cited matters do not change due to the existence of more than the cited matters, but excludes the embodiments of the prior art.

In the entire application document, the term "about" refers to a value that includes the standard deviation of the error of a device or method used to determine the value, represents replaceable features that can be reasonably expected within the listed range, and specifically refers to a given quantity, such as means including a deviation of plus or minus five percent.

Although the disclosed content supports the definition of the term "or" as an alternative only and "and/or", unless explicitly stated as an alternative only or mutually exclusive between alternatives, the term "or" in the claims refers to "and/or".

Herein, the terms "optionally", or "optional", or "optional" typically refer to the event or situation that subsequently may but may not necessarily occur, and the description includes circumstances where the event or situation occurs, as well as circumstances where the event or situation does not occur.

The terms "identity" and "homology" described in the present invention refer to that those skilled in the art can adjust the sequence according to actual work needs in the aspects of using amino acid sequences or nucleotide sequences, so that the used sequence has (comprising but not limited to) 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, 99.9% similarity compared to the sequences obtained in the prior art and remains the same function as the original amino acid sequences or nucleotide sequences.

The term "variant" used with regard to peptides or polypeptides refers to peptides or polypeptides whose amino acid sequences differ due to insertion, deletion, or conservative substitution of amino acid(s), but retain at least one biological activity. Variants can also refer to proteins having amino acid sequences that are essentially identical to a reference protein, where the reference protein has an amino acid sequence that retains at least one biologically activity. Conservative substitution of amino acid(s), i.e. substituting amino acid(s) with different amino acid(s) that has similar properties (such as hydrophilicity, degree and distribution of charged regions), is considered in the art as a typically involving minor change. As understood in the art, these minor changes can be partially identified by considering the hydrophilicity index of amino acid(s). (Kyte et al., 1982, J. Mol. Biology. 157:105-132). The hydrophilicity index of amino acid(s) is based on their hydrophobicity and charge considerations. It is known in the art that amino acid(s) with similar hydrophilic indices can be substituted while still retaining protein function. On the one hand, amino acid(s) with a hydrophilic index of + 2 are substituted. The hydrophilicity of amino acids can also be used to reveal substitution(s) that leads to retention of biological functions of a protein. Considering the hydrophilicity of amino acids in the context of a peptide allows for calculation of the maximum local average hydrophilicity of the peptide, which is a useful measurement reported to be closely related to antigenicity and immunogenicity. US Patent No. 4,554,101, which is incorporated herein in its entirety by reference. Substituting amino acid(s) with similar hydrophilicity value(s) can result in a peptide that retains biologically activity s, such as immunogenicity, as understood in the art. Amino acids of hydrophilic values with a difference within ± 2 between each other can be used for substitution. The hydrophobicity index and hydrophilicity value of an amino acid are both influenced by the specific side chain of the amino acid. Consistent with this observation, amino acid substitutions that are compatible with biological functions are understood as depending on a relative similarity of amino acids, especially the side chains of those amino acids, such as hydrophobicity, hydrophilicity, charge, size, and other properties.

The term "variant" as used herein regarding nucleic acids refers to (i) a portion or a fragment of a reference nucleotide sequence; (ii) a complementary sequence of a reference nucleotide sequence or portion thereof; (iii) a nucleic acid that is substantially identical to a reference nucleic acid or complementary sequence thereof; (iv) a nucleic acid that hybridizes under strict conditions with a reference nucleic acid, complementary sequence thereof, or a sequence that is substantially identical thereto. The variant can be a nucleic acid sequence that is substantially identical in a full length of an entire gene sequence or fragments thereof. A nucleic acid or a protein sequence can be 66%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100% identical in a full length of a gene, protein sequence or fragment thereof.

Herein, the term "identity", "homology", or "similarity" , when used to describe an amino acid sequence or a nucleic acid sequence relative to a reference sequence, a percentage of identical amino acids or nucleotides between two amino acid sequences or nucleic acid sequences is determined by conventional methods, for example, see Ausubel et al. (1995), Current Protocols in Molecular Biology, Chapter 19 (Greene Publishing and Wiley-Interscience, New York); and the ALIGN program (Dayhoff (1978), Atlas of Protein Sequence and Structure 5: Suppl. 3 (National Biomedical Research Foundation, Washington, D.C.). There are many algorithms for aligning sequences and determining sequence identity, including the homology alignment algorithm proposed by Needleman et al. (1970), J. Mol. Biol. 48:443; the local homology algorithm proposed by Smith et al. (1981), Adv. Apply. Math. 2:482; the similarity search method proposed by Pearson et al. (1988) ), Proc. Natl. Acad. Sci. 85:2444; Smith Waterman algorithm (Meth. Mol. Biol. 70:173-187 (1997); and BLASTP, BLASTN, and BLASTX algorithms (see Altschul et al. (1990) J. Mol. Biol. 215:403-410). Computer programs utilizing these algorithms are also available, including but not limited to ALIGN or Megalign (DNASTAR) software, or WU-BLAST-2 (Altschul et al., Meth. Enzym., 266: 460-480(1996)); or GAP, BESTFIT, BLAST Altschul, etc., as mentioned above, FASTA, and TFASTA, available in the Genetics Computing Group (GCG) package, version 8, Madison, Wisconsin, USA; CLUSTAL in the PC/Gene program provided by Intelligentics, Mountain View, California.

"Hybridization or hybridizing" refers to the formation of double stranded molecules by pairing between complementary polynucleotide sequences (such as the genes listed in Tables 1 and 2) or portions thereof under different strict conditions. (See, for example, Wahl, GM. and S.L. Berger (1987) Methods Enzymol. 152:399; Kimmel, A.R. (1987) Methods Enzymol.152: 507).

### General definitions of terminologies in genetic engineering and cell engineering

The term "cell" refers to a naturally occurring or modified intact living cell. Cells can be separated from other cells, mixed with other cells in the culture, or mixed in tissues (partial or entire) or organisms. The method described herein can be performed on a sample comprising a single cell, a population of cells s, or a tissue or an organ containing cells.

As used herein, the term "mammalian cell" refers to any cell derived from a mammalian subject that is suitable for transplantation into the same or different subjects. Cells can be heterogeneous, autologous, or allogeneic. Cells can be primary cells obtained directly from mammalian subjects. Cells can also be cultured and expanded cells derived from cells obtained from the subject. In some embodiments, cells have been genetically engineered to express recombinant protein(s) and/or nucleic acid(s).

Vectors and recombinant vectors are used to carry target genes. In the present invention, lentiviral vector (LV) is preferred, which is an RNA virus, a type of retroviral viruses, which can effectively integrate exogenous gene(s) into host chromosome for persistent expression. In the present invention, Adeno-associated viral vector (AAV) is preferred, which is a non-integrated DNA virus, and widely used in gene therapy due to its higher safety, lower immunogenicity, and longer *in vivo* expression time. Engineered mRNA, including linear mRNA and circular mRNA, is a novel gene vector suitable for non-viral gene delivery systems and can be used for *in vivo* gene therapy. Circular mRNA is preferred in the present invention as it has lower immunogenicity and higher stability than linear mRNA, making it suitable for long-term expression of therapeutic proteins *in vivo.* Therefore, it is increasingly applied in gene therapy. But any vector that can be used for carrying is within the protection scope of the present invention.

Host cells include dividing cells and non-dividing cells; they can effectively infect primary cells, neuronal cells, glial cells, stem cells, mesenchymal stem cells, myocardial cells, liver cells, T cells, tumor cells, etc., as well as fibroblasts, epithelial cells, blood cells, immune cells, nerve cells, embryonic cells, etc. More specifically, such as human skin fibroblasts, for example, HFF-1, HSF-1, HDF, BJ, etc; such as human skin keratinocytes, for example, HACAT and the like; such as human T cells, for example, Jurkat, and the like; such as human lung fibroblasts, for example IMR-90 and the like.

### Definition of terminologies in the field of reprogramming

The term "rejuvenated cells" refers to aged cells that have been treated or transiently reprogrammed with one or more cell reprogramming factors, making the cells possess transcriptomic features of young cells while still retaining one or more cell identity markers.

The term "stem cells" refers to cells that retain the ability to renew themselves through mitotic cell division and can differentiate into a diverse range of specialized cell types. Totipotent stem cells are produced by the fusion of an egg and a sperm cell. The cells produced by the first few divisions of a fertilized egg are also totipotent. These cells can differentiate into embryonic and extra embryonic cell types. Embryonic stem cells are the offspring of totipotent cells. Embryonic stem cells are pluripotent and capable of differentiating into cell types from any of the three germ layers. Induced pluripotent stem cells (iPSCs) are derived from adult cells, which are induced into embryonic stem cell like pluripotent states by cell reprogramming factors. Induced pluripotent stem cells can be derived from adult somatic cells such as skin or blood cells, and also have the potential to differentiate into any of the three germ layer cell types.

As used herein, the term "reprogramming" refers to the use of cell reprogramming factors to induce somatic cell conversion into iPSCs. The reprogramming process can be divided into three phases: the initiation phase, the maturation phase, and the stabilization phase. In the initiation phase, cells still maintain their original somatic identity, but the expression of early pluripotency related genes such as NANOG and SALL4 rapidly increase; in the maturation phase, somatic cell identity features art rapidly lost, and the expression of late phase pluripotency related genes such as DPPA5 and LIN28 increases; and in the stabilization phase, cells completely lose their cell identity and become iPSCs. In addition, this process can also restore the vitality of aged cells (i.e. reduce all or some aging markers).

As used herein, the term "loss of cell identity" refers to the reprogramming of somatic cell to lose the unique morphologies, functions, epigenetic patterns, gene expression profiles, and other characteristics of the original somatic cells, to obtain various characteristics of pluripotent stem cells and become iPSCs. It should be noted that the cell identity of somatic cells is gradually lost in the process of reprogramming. In the initiation phase, the cells still maintain their original cell identity; and in the maturation phase, the cells have lost most of their cell identity characteristics and have acquired a high degree of pluripotent stem cell characteristics. At this point, even if reprogramming is stopped, there is still a great risk that the cells cannot restore their original cell identity.

The term "partial reprogramming" refers to the treatment or transient reprogramming of an aged cell using one or more cell reprogramming factors to restore viability of the cells to certain extent but not enough to dedifferentiate into iPSCs, and retain their original cell identity. "Transient reprogramming" is an implementation of partial reprogramming, which refers to the exposure of cells to cell reprogramming factors for a sufficient period of time to restore the vitality of cells, but not enough to cause dedifferentiation into iPSCs. Due to the very short exposure time, the degree of vitality restoration of such transient reprogramming is relatively low.

Initiation phase reprogramming refers to modifying cell reprogramming factors through engineering to stagnate the reprogramming of an old cell in the initiation phase, thereby providing a longer safe time window to restore vitality and greatly reducing the risk of becoming iPSCs. The main difference between it and transient reprogramming lies in the engineering modification of cell reprogramming factors, which allows for manual control of the phase process of reprogramming at the molecular level.

The term "liquid-liquid phase separation" (LLPS), also known as phase separation, refers to a process in which certain proteins or nucleic acid molecules within a cell can interact with each other through multivalent interactions to produce another phase with different physical and chemical properties in the surrounding originally homogeneous liquid environment, typically appear as droplets at micron level in size (similar to oil droplets in water), which form a relatively independent space that selectively enriches certain protein molecules, resulting in higher protein density and reduced molecular micro motion. They can promote certain biochemical reaction results, such as transcriptional regulation of genes, changes in chromosome spatial structure, and so on. There are many mechanisms for protein phase separation, one of which relies on the intrinsically disordered domain (IDR) of a protein. The acidic polar amino acids in this domain carry negative charges and can interact with each other to form phase separation. Another type of mechanism relies on the ubiquitination of lysine, through the interaction of multiple ubiquitination chains, phase separation can also be formed. "Phase separation-deficient or deficient in phase separation" refers to a mutation of a IRD sequence or key amino acid(s) that forms phase separation, causing variant proteins to be unable to form LLPS, thereby affecting (reducing) their intracellular biological functions.

As used herein, the term "cell reprogramming factor" refers to a set of transcription factors and combinations thereof that can convert adult or differentiated cells into pluripotent stem cells. Specifically, reprogramming factors are factors from Oct family, Sox family, Klf family, Myc family, Nanog family, Glis family, or Lin family. Exemplary reprogramming factors comprise OCT4, SOX2, KLF4, c-MYC, LIN28, NANOG, and/or GLIS1. Other exemplary reprogramming factors comprise CMYC, DPPA2, DPPA4, ESRRB, GDF3, GLIS1, KLF2, KLF4, KLF5, LIN28, LMYC, NANOG, NMYC, NR5A1, NR5A2, OCT-4, RCOR2, SALL1, SALL4, SOX1, SOX2, SOX3, TDRD12, TET1, TH2A, TH2B, UTF1, ZFP42, MDM2, CyclinD1, SV40 large T antigen, SIRT6, TCL1A and RARy. Wherein the "Yamanaka factors" include, but not limit to, the combination of four transcription factor genes Oct-4, Sox4, Klf4 and c-Myc (collectively referred to as OSKM genes), in some aspects may also be the genes Oct4, Sox-2 and K1f4 (OSK genes), in some aspects may also be the genes Oct4, Klf4 and c-Myc (OKM genes), and in some aspects may also be the genes Sox-2, Klf4 and c-Myc (KMS genes).

Oct reprogramming factor refers to any naturally occurring member of the transcription factor octamer family, or variant thereof that maintains transcription factor activity, a naturally occurring family member that is similar (within at least 50%, 80%, or 90% of the activity range) to the closest related factor, or a polypeptide that at least contains a DNA binding domain of a naturally occurring family member, and may further include a transcription activation domain. Exemplary Oct polypeptides comprise Oct-1, Oct-2, Oct-3/4, Oct-6, Oct-7, Oct-8, Oct-9, and Oct-11, such as Oct3/4 (referred to as "Oct4" herein) containing a POU domain. In some embodiments, variants have at least 85%, 90%, or 95% amino acid sequence identity across their whole sequence compared to naturally occurring members of the Oct polypeptide family. In some embodiments, the OCT4 reprogramming factor protein/polypeptide provided herein is encoded by an optimized polynucleotide sequence SEQ ID NO: 1. Therefore, compared to the wild-type OCT4, SEQ ID NO: 1 constitutes an altered polynucleotide sequence. Compared to an amino acid sequence SEQ ID NO: 2, the amino acid sequence encodes a more robust OCT4 reprogramming factor in some embodiments. In some embodiments, the OCT4 reprogramming factor protein/polypeptide is encoded by a polynucleotide or an amino acid sequence having at least about 80%, about 85%, about 90%, about 95%, about 96%, about 97%, about 98%, about 99% identity.

"Sox family" refers to genes encoding SRY (sex determining region Y)-box 2, also known as SOX2, which are associated with maintaining pluripotency. Sox polypeptides (such as Sox1, Sox2, Sox3, Sox15, or Sox18) can be derived from humans, mice, rats, cows, pigs, or other animals. Therefore, the terms "protein" and "SOX2 protein" and so on referred to herein include any natural form of Sox2 transcription factor or its maintenance of activity of Sox2 transcription factor (e.g., at least 50%, 80%, as measured by methods known in the art, with 90% or 100% activity compared to wild-type Sox2). In some examples, variants have at least 90% amino acid sequence identity across their whole sequence compared to naturally occurring Sox2 polypeptides. Specifically, sequence improvement with identity control is carried out based on SEQ ID NOs: 23-24.

"K1f family" refers to Kruppel like factor 4 or a "Klf" gene encoding a Klf4 protein, which is a protein encoded by mouse klf4 gene and human klf4genes. Exemplary Klf family members include Klf1, Klf2, Klf3, Klf-4, Klf5, Klf6, Klf7, Klf8, Klf9, Klf10, Klf11, Klf12, Klf13, Klf14, Klf15, Klf16 and Klf17. Therefore, the terms "KLF4", "KLF4 protein", etc. referred to herein include any naturally occurring form of KFF4 transcription factor or its maintenance of KLF4 transcription factor activity (e.g., at least 50%, compared to wild-type KFF4, 80%, 90%, or 100% activity, as measured by methods known in the art). In some embodiments, variants have at least 85%, 90%, or 95% amino acid sequence identity across their whole sequence compared to naturally occurring KFF4 polypeptides. For example, the variant structure shown in SEQ ID NO: 21-22.

Factors of the Myc family refer to factors encoded by myc proto-oncogene implicated in cancer. N-Myc or L-myc can also been used as a potential reprogramming factor to replace c-Myc. Therefore, the terms "c-Myc", "C-MYC", "c-Myc protein", "C-MYC protein", etc. referred to herein include any naturally occurring form of the cMyc transcription factor or variant thereof that maintains cMyc transcription factor activity (e.g., within at least 50%, 80%, 90%, or 100% activity compared to wild-type cMyc, as measured by methods known in the art). Specifically, variant improvements can be made based on the wild-type sequence of SEQ ID NO: 11-12.

In one embodiment of this aspect and all other aspects described herein, the reprogramming comprises one of the following: introducing nucleic acid sequences encoding transcription factors Oct4, Sox2, c-Myc, and Klf4, etc. into the differentiated somatic cells, the sequences are operatively linked to regulatory element(s) for expressing the factors; importing one or more protein factors that reprogram the cell differentiation state; and contacting the cells with small molecules that induce reprogramming of the differentiation state of the cells.

### Variant

The present invention also includes fragments, derivatives, and analogs of transcription factor mutants. The terms "fragment", "derivative", and "analog" refer to proteins that essentially maintain the same biological function or activity as the transcription factor mutant of the present invention. The protein fragments, derivatives, or analogs of the present invention can be (i) proteins with one or more conserved or non-conserved amino acid residues (preferably conserved amino acid residues) substituted, and such substituted amino acid residues may or may not be encoded by the genetic code, or (ii) proteins with substituent group(s) in one or more amino acid residues, or (iii) proteins formed by fusing additional amino acid sequences to this protein sequence (such as leader sequences or secretory sequences or sequences used to purify this protein or proprotein sequences, or fusion proteins). As defined herein, these fragments, derivatives, and analogues are within the scope known to those skilled in the art. However, in the amino acid sequences of the transcription factor mutants and their fragments, derivatives, and analogues, corresponding to the wild-type sequence, there must be a mutation at any of the positions described in the preceding paragraph of the present invention.

### Definition of terms in the field of drug therapy

Herein, the term "treatment", "treat" or "treating" refers to obtaining desired pharmacological and/or physiological effects. The effects may be preventive in terms of completely or partially preventing a disease or its symptoms, and/or therapeutic in terms of partially or completely curing a disease and/or adverse effects caused by the disease. The term "treatment", "treat" or "treating" used herein encompasses diseases in mammals, especially cats, comprising: (a) preventing the occurrence of a disease or condition in an individual who is susceptible to the disease but has not yet been diagnosed with the disease; (b) inhibiting a disease, such as blocking disease progression; or (c) alleviating a disease, such as reducing symptoms associated with the disease. The term "treatment", "treat" or "treating" used herein encompasses any medication in which a medicament or a compound is administered to an individual to treat, cure, alleviate, improve, reduce, or inhibit a disease in the individual, including but not limited to administering a medicament containing the compound described herein to an individual in need thereof.

The terms "object", "individual", "patient", or "subject" include (selecting addition depending on whether there is a therapeutic use) mammals. Mammals include but are not limited to domesticated animals (such as cows, sheep, cats, dogs, and horses), primates (such as humans and non-human primates such as monkeys), rabbits, and rodents (such as mice and rats).

The term "prevention", "prevent" or "preventing" referred to in the present invention refers to all behaviors of avoiding symptoms or delaying specific symptoms by administering the product described in the present invention before or after the onset or development of a disease.

The term "effective dose" referred to in the present invention refers to an amount or dosage of the product described in the present invention that provides the desired treatment or prevention after being administered to a patient or organ in a single or multiple doses.

The term "pharmaceutical composition" herein typically refers to a unit dose form and can be prepared using any method well-known in the pharmaceutical field. All methods include the step of combining an active ingredient with a carrier that constitutes one or more additional ingredients. Generally a composition is prepared by uniformly and sufficiently combining an active compound with a liquid carrier, finely divided solid carrier, or both.

The term "gene therapy" refers to transient or permanent genetic modification (such as gene expression, insertion, alteration, or removal) of individual cells and/or biological tissues to treat diseases, such as tumors, genetic diseases, senescence-associated diseases, and autoimmune diseases. The most common form of gene therapy involves introducing functional gene(s) into cells for expression to treat diseases, inserting functional gene(s) into specific or non-specific genomic position(s) to substitute mutated gene(s), while other forms include directly correcting mutation(s) or modifying normal gene(s) that is susceptible to pathogen infection, or transferring gene(s) or gene fragment(s) into cells for transcription.

The term "cell therapy" refers to a pharmaceutical product that achieves therapeutic, diagnostic, and preventive purposes through a series of actions, such as proliferating and screening live autologous, allogenic, or xenogenic cells in vitro to restore cell and tissue function, or altering cellular biological characteristics in other ways.

### The first aspect of the present invention relates to a cell rejuvenation method

The inventor studied the mechanism of three stages of cell reprogramming, initiation phase: cell identity continues to be maintained, early-stage pluripotency genes are upregulated, epigenetic age (epiAge) decreases, and cells begin to rejuvenate; maturation phase: cell identity is dramatically lost, late-stage pluripotency genes are upregulated; stabilization phase: the cell identity is completely lost and becomes an iPSC. Therefore, there is a time window for partial reprogramming (PR) before the maturation phase. The inventor has discovered a new method of preventing cell reprogramming from entering the maturation phase, extending the PR time window, and rejuvenating cells without losing cell identity. The method involves using engineered phase separation-deficient reprogramming factors, based on mutations in the phase separation function related regions of transcription factors, to cause transcription factors to lose their ability to trigger phase separation, resulting in their inability to drive cell reprogramming into the maturation phase. This causes reprogramming to remain in the initiation phase, continuously rejuvenating cells but greatly reducing the risk of loss of cell identity due to entrance of the reprogramming into the late stage. This method is called "initiation phase reprogramming".

Furthermore, the phase separation- deficient engineered transcription factors can be OCT4, SOX2, KLF4, c-MYC, L-myc, LIN28, NANOG, and GLIS1 and the like.

The present invention relates to engineering modification of transcription factors to make them lose their phase separation ability. The inventor found that the modification and improvement of the three-dimensional structure of transcription factors do not need to be limited, as long as they lose their phase separation ability and retain cell reprogramming in the initiation phase, it will be within the protection scope of the present invention.

Therefore, the present invention relates to a method for cell initiation phase reprogramming mediated by phase separation-deficient engineered transcription factors, characterized in that, it is achieved by introducing phase separation-deficient engineered transcription factors into cells.

A method of rejuvenating cells, characterized in that, phase separation-deficient engineered transcription factors are used to mediate cell initiation phase reprogramming.

A method of cell reprogramming, characterized in that, it is achieved by introducing a vector containing phase separation-deficient engineered transcription factors into cells.

Specifically, criteria for judging cell rejuvenation is that rejuvenated cells exhibit changes in gene expression of one or more epigenetic markers compared to a reference value, the changes are either an increase or a decreases.

Specifically, criteria for judging cell rejuvenation is that rejuvenated cells exhibit a change in epigenetic age (epiAge) of DNA methylation compared to a reference value, the change is a decrease.

Specifically, criteria for judging cell rejuvenation is, for example, a change in the proportion of the rejuvenated cells exhibiting senescence-associated β-galactosidase (SA - β-Gal) positivity compared to a reference value, the change is a decrease.

Specifically, examples of criteria for judging cell rejuvenation include: detecting genes related to rejuvenation function, such as COL1A1, COL3A1, COL4A1, FN1, and LAMA5, with the expression levels of the genes being increased relative to reference values; detecting feature genes in the initiation phase of reprogramming: NANOG, SALL4, with the expression levels of genes being increased; detecting feature genes of reprogramming maturation phase: DPPA5, LIN28A, with expression levels of the genes decreased. Cell identity genes can be used and detected for cell validation, such as detecting fibroblast identity genes: THY1, P4HB, S100A4, SERPINH1; selecting different identity genes based on different cells; after testing, the expression of identity gene(s) can be increased relative to reference value(s).

The selection of the reference values, it can be blank cell controls of the same cell type, or wild-type transcription factor recombinant vectors introduced into cells as controls. In short, a reference value is not an absolute standard, but a relative comparison benchmark for modified transcription factor recombinant vectors introduced into cells for comparison. Therefore, the above "criteria for determining rejuvenation" are only examples and not absolute standards. As long as it can be proven that cell reprogramming remains in the initiation phase, it will be within the scope of the criteria for determining cell rejuvenation.

The scope of protection of the present invention is not limited to the exemplary functional genes mentioned above. As long as it can be used to detect and determine that partial reprogramming of cells remains in the initiation phase, it will be within the scope of protection of the present invention.

It is worth noting that methods of cell rejuvenation or cell reprogramming can be used for treatment or diagnosis, or may not be used for treatment or diagnosis. They can be used to treat in vivo or ex vivo cells, and may not necessarily be directly applied to the subject in need of treatment. In a specific embodiment, the method of cell rejuvenation or cell reprogramming is not used for the diagnosis, prevention, and treatment of diseases.

The specific engineering/mutation/modification methods for element modification of the phase separation-deficient engineering transcription factor can be as follows, and the following improvement methods are only examples:
(1) Mutation(s) of charged amino acid(s), including mutating acidic charged amino acid(s) and/or alkaline charged amino acid(s), to change charged amino acid(s) in an intrinsically disordered domain (IDR region) of a protein to regulate its ability to form condensates.

The inventor discovered that one of the principles is that "negative charged IDR causes phase separation of a transcription factor". Therefore, by predicting a internal IDR region of a transcription factor, such as using VSL2 algorithm to predict a IDR of OCT4 through Disorder Score, for example, continuous regions with scores greater than a certain value have a high probability of being IDR. The value is not particularly limited, but can be in a range of 0.4-0.6, preferably 0.5. The exemplary prediction results for OCT4 show that the IDR of OCT4 is distributed in Activation Domain (AD) at both ends, more specifically at amino acids positions 1-140 and positions 288-360; the intermediate DNA binding domain (DBD) does not belong to IDR. It is worth noting that alternative scoring criteria can also be used, such as the scoring criteria of IUPred2 and ANCHOR2, but as long as it can be used for predictive scoring in a IDR region, it will be within the scope of protection of the present invention. The inventor predicted that the conclusions obtained from multiple scoring criteria are the same (within the error range), therefore the present invention can protect an IDR region structure predicted and obtained by multiple scoring criteria.

Specifically, amino acid modification(s) can be made to an intrinsically disordered domain (IDR region) of a transcription factor protein, or to an activation domain. Furthermore, charged amino acid(s) can be modified, such that an ability of phase separation of transcription factor(s) is removed.

Specifically, a polar charged amino acid can be substituted with a neutral amino acid, and the polar charged amino acid can be an acidic amino acid.

Preferably, a polar charged amino acid in a IDR region can be substituted with a neutral amino acid, and the polar charged amino acid can be an acidic amino acid.

More specifically, acidic amino acids such as aspartic acid (Asp) and glutamic acid (Glu) in an IDR region can be mutated into neutral amino acids such as alanine.

More specifically, acidic amino acids such as aspartic acid (Asp) and glutamic acid (Glu) in the IDR region can be mutated to neutral amino acids such as alanine.

In a specific example, a wild-type amino acid sequence of OCT4 may be mutated and improved; the IDR region of the protein sequence of the OCT4 wild-type gene can account for 38-40% of the total sequence at the N-terminus and 19-24% at the C-terminus.

Preferably, above mutation and improvement can be made at positions 1-140 of SEQ ID NO: 2, and/or on positions 288-360 of SEQ ID NO: 2. It is worth noting that there may be a margin of error of about 10-20 amino acid positions before and after the above positions.

Preferably, the mutation site is selected from the group consisting of 8, 20, 31, 108, 138, 291 and 297 of SEQ ID NO: 2; at least one of 26, 56, 68, 91, 96, 98, 104, 113, 125, 127, 130, 134, 135, 296, 299, 341 and 343; preferably, the mutation site is selected from at least one of 145, 147, 166, 188, 209, 210, 215, 224, 238, 246, 270 and 272 of SEQ ID NO: 2. Specifically, the number of mutations can be 1, 2, 3... 36.

Exemplarily, the wild-type OCT4 gene can be mutated, such that the acidic polar amino acids in the IDR region of its protein (D/E→A) is mutated, hereinafter referred to as OCT4'; for example, a sequence shown in any of SEQ ID NOs: 4-10, or a sequence with at least 85%, 90%, or 95% or above identity that can achieve the same function.

The present invention further protects nucleotide sequence(s) expressing the amino acid sequence(s) of the above-mentioned protein, exemplarily, which can be shown as SEQ ID NO: 3. However, those skilled in the art know that as long as a target protein can be expressed through codons, different nucleotide sequences obtained by changes of reasonable codon preference are within the scope of protection.

Of course, the present invention is not limited to the definitions of above-mentioned transcription factors, and any transcription factor modification that can achieve z phase separation-deficient function by modifying charged amino acid(s) in an IDR region is within the scope of protection of the present invention.

### (2) Ubiquitination modification

The inventor found that after an amino acid is modified by a polyubiquitin chain, polyubiquitin chain mediated phase separation occurs. Therefore, substituting lysine K with arginine R, for example, does not produce polyubiquitin chain modification and can avoid phase separation. However, it is worth noting that the scope of protection of the present invention is to provide a method for ubiquitination mutation of a transcription factor to avoid phase separation, and is not limited to specific mutation modes.

Exemplarily, for the C-MYC transcription factor, its lysine that can be ubiquitinated is mutated (K→R), and other transcription factors can also be mutated for ubiquitination.

Specifically, SEQ ID NOs: 11-12 can be mutated. Based on SEQ ID NO: 12, at least one lysine (K) in positions 51, 52, 126, 143, 148, 157, 206, 269, 275, 289, 298, 317, 323, 326, 341, 355, 371, 389, 392, 397, 398, 412, 422, 428, and 430 can be mutated; preferably, 1, 2, 3,... 25 of the above positions can be mutated; preferably, one or both of amino acids at positions 148 and 389 are mutated; preferably, 20-25 positions are mutated; more preferably, 25 positions are mutated. However, as long as the removal of ubiquitination chains can be achieved and the desired effect can be achieved, that is, avoiding phase separation, the number of specific mutation positions does not need to be particularly limited.

Preferably, modification method is to mutate lysine K to arginine; but the specific method is not limited, as long as it can remove ubiquitination and avoid phase separation, it is within the scope of protection.

For example, the variants as shown in SEQ ID NOs: 14-20 can be used as examples, or sequences with at least 85%, 90%, or 95% or above identity that can achieve the same function.

The present invention further protects a nucleotide sequence expressing an amino acid sequence of the aforementioned protein, exemplarily, which can be the sequence shown in SEQ ID NO: 13, or a sequence having at least 85%, 90%, or 95% or more identity that can achieve the same function; However, those skilled in the art know that as long as a target protein can be expressed through codons, different nucleotide sequences obtained by reasonable codon preference changes according to the needs of expressing cells will be within the scope of protection.

Of course, the present invention is not limited to the definitions of above-mentioned transcription factors, and any transcription factor modification that can achieve a phase separation-deficient functional removing ubiquitination is within the scope of protection of the present invention.

(3) Partial deletion treatment, preferably a terminal deletion treatment, can involve partial deletion treatment to the N-terminus or C-terminus, or partial deletion treatment to the middle portion of a gene; or referred to as partial deletion of a IDR region of a protein, i.e. partial deletion of charged amino acid(s) in a IDR region; or referred to as a deletion treatment of phase separation critical regulatory region in a protein, namely, a deletion treatment of critical lysine with ubiquitination chain modification, or a deletion treatment of an amino acid that plays a critical role in formation of phase separation.

Exemplarily, a deletion treatment may be made at the N- or C-terminus, or at non-critical regions outside of its DNA-binding domain (DBD) of a transcription factor, e.g., a deletion treatment at positions 1-20, further, a deletion at positions 2-20, 1-19, 2-29, 2-18, 2-17, 2, or 16. Such treatments do not affect the DBD region, that is, do not influence the original DNA site recognition function of a transcription factor, but can remove its IDR region, ubiquitin chain modified lysine(s), or other critical amino acid(s), thereby avoiding phase separation.

Specifically, a partial deletion may be made to a sequence of KLF transcription factor 4 (KLF4) shown in SEQ ID NOs: 21-22 , for example, since its critical DBD region is located at positions 375-470 at the C-terminus, a partial or complete deletion treatment may be made to the IDR region at positions 2-374 at the N-terminus, to obtain, for example, an amino acid sequence shown in SEQ ID NO: 40 or a sequence with at least 85%, 90%, 95% or more identity that can achieve the same function.

Specifically, a partial deletion may be made to a sequence of sex-determining region Y-box 2 (SOX2) transcription factor shown in SEQ ID NOs: 23-24, for example, since its critical DBD region and transcription activation domain are located at positions 41-200 in the middle portion, a complete or partial deletion may be made to the IDR regions at positions 2-40 at the N-terminus and/or positions 201-317 at the C-terminus, to obtain , for example, an amino acid sequences shown in SEQ ID NOs: 41-43, or a sequence with at least 85%, 90%, 95% or more identity that can achieve the same function.

Specifically, a partial deletion may be made to a sequence of OCT4 shown in SEQ ID NOs: 1-2, for example, since its critical DBD region is located at positions 141-287 in the middle portion, a complete or partial deletion may be made to the IDR regions at positions 2-140 at the N-terminus and/or positions 288-360 at the C-terminus to obtain, for example, an amino acid sequences shown in SEQ ID NOs: 44-46, or a sequence with at least 85%, 90%, 95% or more identity that can achieve the same function.

Specifically, a partial deletion may be made to a sequence of c-Myc shown in SEQ ID NOs: 11-12, for example, since its most critical DBD is a bHLH-LZ domain at positions 361-439 at the C-terminus, a complete or partial deletion at positions 2-360 at the N-terminus may be made to obtain, for example, an amino acid sequences shown in SEQ ID NOs: 47-48, or a sequence with at least 85%, 90%, 95% or more identity that can achieve the same function. The present invention further protects nucleotide sequence(s) expressing amino acid sequence(s) of above protein(s), exemplarily, which may be shown in SEQ ID NO: 13, or a sequence having at least 85%, 90%, 95% or more identity that can achieve the same function. However, those skilled in the art know that as long as a target protein can be expressed through codons, and different nucleotide sequences obtained by reasonable codon preference changes are within the scope of protection.

Of course, the present invention is not limited to definitions of the above-mentioned transcription factors, and any transcription factor modification that can achieve no phase separation by a partial deletion treatment is within the scope of protection of the present invention.

### The second aspect of the present invention provides an element comprising engineered transcription factor(s)

### Nucleic acid element

The present invention further comprises nucleic acid element(s) comprising the aforementioned engineered transcription factor(s), the element can be a DNA element, an RNA element, and further can be a mRNA element, a siRNA elements a circular RNA element, a circular mRNA element, a tRNA element.

The element further comprises a Kozak sequence, a promoter sequence, at least one linker peptide sequence, a UTR region, a terminator sequence, a 5' cap, a polyA tail, etc; the nucleic acid element can be a DNA sequence, an RNA sequence, or a further expressed protein element. The Promoter can be selected from an inducible promoter such as TRE, TRE3G, TRE3GS, TRE3GV, etc., a constitutive promoter such as CMV, EFS, CAG, PGK, etc., a post-transcription regulatory sequence such as WPRE etc., and a poly (A) tail such as SV40 poly (A), pGH poly (A), etc.

The mRNA element includes but is not limited to linear mRNA and circular mRNA. IRES and self-cleaving polypeptide 2A elements may be present on the circular mRNA. The RNA may have moderate modifications, including but not limited to pseudouridine (Ψ), N1-methyl-pseudouridine (m1Ψ), 5-methoxyuridine (5moU), N6-methyl-adenosine (m6A), 5-methyl-cytidine (5m5C), etc. There may be sequence(s) on the RNA that bind to L7Ae, TAT, etc. required for enrichment of the RNA.

### Recombinant vector element

The elements in the present invention can further construct a vector, which can be monocistron, bicistron or multicistron. In a specific embodiment of the present invention, a coding region of the bicistronic or multicistronic gene can be linked by at least one linker peptide, which can be an internal ribosome entry site (IRES) or a self-cleaving 2A peptide (2A).

The IRES sequences that can be used include but are not limited to: small RNA virus (such as FMDV), Pestivirus (such as CFFV), poliovirus (such as PV), encephalomyocarditis virus (such as ECMV), Aphthovirus (such as FMDV), hepatitis C virus (such as HCV), classical swine fever virus (such as CSFV), mouse corneal leukoplakia virus (such as MLV), simian immunodeficiency virus (such as SIV), or cricket paralysis virus (such as CrPV) and so on. The coding region can also be segmented through "2A linker", the "2A linker" is a short peptide (18-25 amino acids) derived from a virus, also known as a "self-cleaving" peptide, capable of producing multiple proteins from a transcript, including but not limited to P2A/T2A/E2A/F2A, etc.

When multiple transcription factors are arranged in tandem, the specific sequence can be shown in SEQ ID NOs: 25-27. Of course, the present invention also provides a vector for single transcription factors, which are mixed in certain proportions, such as mixed in 1:1:1:1, 1:2:1:2, 1:2:2:1, and 2:1:1:1. The mixture is also within the scope of protection of the present invention.

The present invention further includes a recombinant vector comprising engineered transcription factors, including but not limited to a cloning vector, an expression vector, a shuttle vector, and a viral vector (such as a lentiviral vector, an adeno-associated virus vector, a poxvirus related vector, etc.).

### Recombinant protein

The present invention further comprises a recombinant protein element obtained by translating and expressing the above nucleic acid element, as well as all nucleotide sequences capable of expressing the above protein element. The protein element maintains its function to a certain extent and can also contain certain variant(s).

### Recombinant cells

The present invention further comprises recombinant cells introduced with the aforementioned vector.

Specifically, the method provided by the present invention can be applied to any type of cell that requires rejuvenation. Cells can be separated from other cells, mixed with other cells in the culture, or mixed in tissues (partial or complete) or living organisms. The method described herein can be performed on samples containing single cells, cell populations, or tissues or organs containing cells. The selection of cells for rejuvenation will depend on the therapeutic effect required for treating age-related diseases or conditions. Cells are mammalian cells. Cells include but are not limited to prokaryotic cells and eukaryotic cells. Further, cells may be mammalian cells, such as humans, monkeys, murine (rats, mice), horses, bovine, sheep, pigs, dogs, cats, etc, for example, CRFK cat kidney cells, PG-4 (S+L -) cat brain cells, F81 cat kidney cells, FCA-S1 cat skin cells, CATK1 cat kidney cells, MDCK (NBL-2) dog kidney cells, SCBN dog duodenal cells, DH82 dog kidney malignant tissue hyperplasia cells, HEK293T, HEK293, BHK, CHO, 3T3, NS0, Hela, HT-1080, PERC6, CAP, HKB-11, Huh-7 etc.. In an example, the cells are human cells. In an example, the cells are derived from elderly subjects.

The method provided by the present invention can be performed on cells, tissues or organs of the nervous system, muscular system, respiratory system, cardiovascular system, skeletal system, reproductive system, integumentary system, lymphatic system, excretory system, endocrine system (e.g. nervous system),(endocrine and exocrine) or digestive system. As described herein, any type of cell can be rejuvenated, including but not limited to epithelial cells (such as squamous, cuboidal, columnar, and pseudo stratified epithelial cells), endothelial cells (such as venous, arterial, and lymphatic endothelial cells), and cells of connective tissue, muscle, and nervous system. Such cells may include but are not limited to epidermal cells, fibroblasts, chondrocytes, skeletal muscle cells, satellite cells, cardiomyocytes, smooth muscle cells, keratinocytes, basal cells, ameloblasts, exocrine cells, myoepithelial cells, osteoblasts, osteoclasts, neurons (such as sensory neurons, motor neurons, and interneurons), neuroglia cells (such as oligodendrocytes, astrocytes, ependymal cells, microglia, Schwann cells, and satellite cells), columnar cells, adipocytes, pericytes, stellate cell, lung cells, blood and immune system cells (such as red blood cells, monocytes, dendritic cells, macrophages, neutrophils, eosinophils, mast cells, T cells, B cells, natural killer cells), hormone-secreting cells, germ cells, interstitial cells, lens cells, photoreceptor cells, gustatory cells, and olfactory cells; and from kidney, liver, pancrea, stomach, spleen, gallbladder, intestine, bladder, lung, prostate, breast, urogenital tract, pituitary cells, mouth, esophagus, skin, hair, nails, thyroid gland, parathyroid gland, adrenal gland, eyes, nose or brain.

Specifically, the cells are selected from fibroblasts, endothelial cells, chondrocytes, skeletal muscle stem cells, keratinocytes, mesenchymal stem cells, and corneal epithelial cells. Specifically, the cells are fibroblasts. Specifically, the cells are endothelial cells. Specifically, the cells are chondrocytes. Specifically, the cells are skeletal muscle stem cells. Specifically, the cells are keratinocytes. Specifically, the cells are mesenchymal stem cells. Specifically, the cells are corneal epithelial cells.

### Introduction and Delivery

The introduction methods include but are not limited to liposome transfection, microvesicle transfection, exosome transfection, electroporation, nanocarrier transfection, and chemical transfection using transfection reagents. Nanocarriers include lipids, polymers, or lipid-polymer hybrids.

A delivery system for delivering the above-mentioned nucleic acid molecules, recombinant vectors containing the nucleic acid molecules, recombinant DNA molecules, mRNA, recombinant proteins, etc. In some embodiments, lipid nanoparticles or "LNP" are used to deliver nucleic acid(s) to cells. As mentioned above, LNP can comprise natural lipids or synthetic lipids, including conjugated lipids or polymers (such as PEGylated lipids). LNP can comprise any one or more of neutral lipids, zwitterionic lipids, ionizable lipids, cationic lipids, and anionic lipids. In some embodiments, LNP comprises natural or synthetic monoacyl or diacyl forms of phosphatidylcholine (PC), phosphatidylglycerol (PG), phosphatidylserine (PS), phosphatidylethanolamine (PE), and phosphatidic acid (PA), or monoacyl, diacyl, triacyl, or tetraacyl forms of cardiolipin. In some embodiments, LNP is a micelle or reverse micelle (reversed micelle). In other embodiments, LNP is a monolayer liposome or a multilayer liposome.

The present invention also relates to other delivery systems, such as a GalNAc conjugate linked delivery system, a liposome LNP nanodelivery system (small molecule ligands, antibodies, and other molecules), virus like particle VLP delivery, a protamine, a high molecular polymer, inorganic nanoparticles, an exosomes, polymer matrix, virus transfection, etc.

### The third aspect of the present invention relates to use of prevention and/or treatment

The nucleic acid elements (DNA, RNA elements), recombinant vectors, recombinant proteins, and recombinant cells involved in the present invention can be used for preventive and/or therapeutic uses in multiple fields, as well as for the use in the preparation of medicaments for treating diseases. The specific diseases to be treated include but are not limited to cell therapy, gene therapy, immune cell proliferation, cancer treatment, senescence-associated disease treatment, etc.

The method involved in the present invention can be used to restore the vitality of cultured cells (e.g., *in vitro* or *ex vivo*) to improve the function and efficacy for cell therapy. The cells used to treat patients can be autologous or allogeneic. Preferably, the cells are derived from a patient or a matching donor. For example, in *ex vivo* therapy, cells are directly obtained from a patient to be treated, transfected with mRNA encoding cell reprogramming factor(s) as described in the present invention, and re-implanted into the patient's body. This type of cells can be obtained, for example, from biopsy or surgical procedures performed on patients. Alternatively, cells that require rejuvenation can be directly transfected *in vivo* with mRNA encoding cell reprogramming factor(s). In some embodiments, such immune cell engineering is performed *ex vivo,* such as in the manufacture of cell therapy products, such as autologous or allogeneic chimeric antigen receptor (CAR) - T, CAR-NK, CAR-M, or CAR-NKT cells.

The method involved in the present invention can be used for gene therapy. "Gene therapy" is used to refer to treating and/or preventing a disease in a mammalian subject (e.g. human) using gene(s) (i.e. a genetic material). In some embodiments, the genetic materials (nucleic acid elements (DNA, RNA elements), recombinant vectors, etc.) involved in the present invention are directly delivered to at least some cells of mammalian subjects. The present invention can be used for any specific method or composition of gene therapy. Gene therapy can be achieved by introducing the aforementioned nucleic acid elements, recombinant vectors, etc. into mammalian cells. Specifically, it can include autologous or *ex vivo* gene therapy. Gene therapy can also be divided into "germ line cell gene therapy" and "somatic cell gene therapy". In the case of "germ cell gene therapy", germ cells, namely sperm or egg cells, are genetically modified. Genetic changes are usually integrated into their genomes. Therefore, the changes caused by treatment can be inherited and transmitted to offspring. This method can be used to treat genetic disorders and genetic diseases. In the case of "somatic gene therapy", therapeutic genes are transferred into the individual's somatic cells. Any modifications and effects will be limited only to that individual and will not be inherited by the individual's descendants or offspring. Specific strategies include but are not limited to methods such as DNA correction, DNA replacement, DNA supplementation, and DNA inactivation.

On the other hand, methods for inducing proliferation of cells, such as immune cells, are provided. In some embodiments, the method comprises exposing cells to mRNA encoding one or more reprogramming factors, whereby the exposure achieves the expression of one or more reprogramming factors in the cells to enhance the proliferation of the cells while retaining their characteristics. In some embodiments, the method of inducing proliferation does not induce failure.

In some embodiments, proliferation is caused by prevention or reversal of failure.

Specifically, the cell rejuvenation therapy of the present invention can be used for "age-related diseases or conditions", specifically referring to any senescence-associated conditions, diseases or disorders such as but not limited to neurodegenerative diseases (such as Alzheimer's disease, Parkinson's disease, Huntington's disease, amyotrophic lateral sclerosis, dementia, and stroke), cardiovascular and peripheral vascular diseases (such as atherosclerosis, peripheral arterial disease (PAD), hematoma, calcification, thrombosis, embolism and aneurysm), eye diseases (such as age-related macular degeneration, glaucoma, cataract, xerophthalmia, diabetes retinopathy, hypopsia), skin diseases (dermal atrophy and thinning, elastic hyperplasia and skin wrinkles, sebaceous hyperplasia or hypoplasia, senile freckles and other pigmentation abnormalities, hair graying, loss or thinning, chronic skin ulcers), autoimmune diseases (such as polymyalgia rheumatica (PMR), giant cell arteritis (GCA), rheumatoid arthritis (RA), crystal arthropathy and spondyloarthropathy (SPA)), metabolic dysfunction in internal medicine (such as adult hypopituitarism, hypothyroidism, apathetic thyrotoxicosis, osteoporosis, diabetes, adrenal insufficiency, various forms of hypogonadism and endocrine malignancies), musculoskeletal diseases (such as arthritis, osteoporosis, myeloma, gout, Paget's disease, bone fracture, bone marrow failure syndrome, ankylosis, diffuse idiopathic skeletal hyperostosis, hematogenous osteomyelitis, muscle atrophy, peripheral neuropathy, multiple sclerosis, amyotrophic lateral sclerosis (ALS), Duchenne muscular dystrophy, primary lateral sclerosis, and myasthenia gravis), digestive system diseases (such as cirrhosis, liver fibrosis, Barrett esophagus), respiratory system diseases (such as pulmonary fibrosis, chronic obstructive pulmonary disease, asthma, chronic bronchitis, pulmonary embolism (PE), lung cancer, and infections), conditions related to cell proliferation, and any other senescence-associated diseases and conditions.

As used herein, the term "disease or condition involving cartilage degeneration" refers to any disease or condition involving cartilage and/or joint degeneration. The term "disease or condition involving cartilage degeneration" includes disorders, conditions, syndromes, diseases, and injuries affecting intervertebral discs or joints (e.g. articulus) in animals (including humans), and includes but is not limited to arthritis, cartilage malformation, spinal joint disease, ankylosing spondylitis, lupus erythematosus, relapsing polychondritis, and Sjogren syndrome. The term "muscle degenerative disease or condition" refers to any disease or condition involving muscle degeneration. This term includes conditions, disorders, and syndromes.

Diseases and injuries affecting muscle tissue, such as but not limited to muscle atrophy, muscle disuse, muscle tear, burn, surgery, peripheral neuropathy, multiple sclerosis, amyotrophic lateral sclerosis (ALS), Duchenne muscular dystrophy, primary lateral sclerosis, myasthenia gravis, cancer, AIDS, congestive heart failure, chronic obstructive pulmonary disease (COPD), liver disease, renal failure, eating disorder, malnutrition, hunger, infection or glucocorticoid treatment.

Diseases related to cell proliferation refer to diseases that occur due to abnormal growth or elongation caused by cell proliferation (Walker, Cambridge Dictionary of Biology, Cambridge University Press: Cambridge, UK, 1990). Proliferative diseases may be related to: 1) pathological proliferation of normal quiescent cells; 2) pathological migration of cells from their normal position (e.g. metastasis of tumor cells); 3) pathological expression of proteolytic enzymes such as matrix metalloproteinases (such as collagenase, gelatinase, and elastase); or 4) pathological angiogenesis, such as proliferative retinopathy and tumor metastasis. Exemplary proliferative diseases include cancer (i.e., malignant tumors), benign tumors, angiogenesis, inflammatory diseases, and autoimmune diseases.

"Cancer" refers to a type of disease characterized by the development of abnormal cells that proliferate uncontrollably and have an ability to infiltrate and destroy normal body tissues. Exemplary cancers include but are not limited to acoustic neuroma; adenocarcinoma; adrenal carcinoma; anal cancer; hemangiosarcoma (such as lymphangiosarcoma, lymphangioendotheliosarcoma, angiosarcoma); appendiceal cancer; benign monoclonal gammopathy; carcinoma of bile duct (e.g. cholangiocarcinoma); bladder cancer; breast cancer (for example, breast adenocarcinoma, breast papillary cancer, breast cancer, breast medullary carcinoma); brain cancer (such as meningioma, glioblastoma, neuroglioma (such as astrocytoma, oligodendroglioma), and neuroblastoma); bronchial cancer; carcinoid tumor; cervical cancer (such as cervical adenocarcinoma); choriocarcinoma; chordoma; craniopharyngeal duct tumor; colorectal cancer (e.g. colon cancer, rectal cancer, colorectal adenocarcinoma); connective tissue cancer; epithelial carcinoma; ependymoma; endotheliosarcoma (e.g. Kaposi sarcoma, multiple idiopathic hemorrhagic sarcoma); endometrial cancer (e.g. uterine cancer, uterine sarcoma); carcinoma of esophagus (e.g. esophageal adenocarcinoma, Barrett adenocarcinoma); Ewing's sarcoma; eye cancer (such as intraocular melanoma, retinoblastoma); familiar eosinophilia; carcinoma of gallbladder; carcinoma of stomach (e.g. gastric adenocarcinoma); gastrointestinal stromal tumor (GIST); germ cell carcinoma; head and neck cancer (such as head and neck squamous cell carcinoma, oral cancer (such as oral squamous cell carcinoma), throat cancer (such as laryngeal cancer, pharyngeal cancer, nasopharyngeal cancer, oropharyngeal cancer); hematopoietic system cancers (e.g. leukemia, such as acute lymphoblastic leukemia (ALL) (e.g. B-cell ALL, T-cell ALL), acute myeloid leukemia (AML) (e.g. B-cell AML, T-cell AML), chronic myeloid leukemia (CML) (e.g. B-cell CML, T-cell CML), and chronic lymphocytic leukemia (CLL) (e.g. B-cell CLL, T-cell CLL)); lymphoma, such as Hodgkin lymphoma (HL) (e.g. B-cell HL, T-cell HL) and non-Hodgkin lymphoma (NHL) (e.g. B-cell NHL, such as diffuse large cell lymphoma (DLCL) (e.g. diffuse large B-cell lymphoma), follicular lymphoma, chronic lymphocytic leukemia/small lymphocytic lymphoma (CLL/SLL), mantle cell lymphoma (MCL), marginal zone B-cell lymphoma (e.g. mucosa associated lymphoid tissue (MALT) lymphoma, lymph node marginal zone B-cell lymphoma, splenic marginal zone B-cell lymphoma), primary mediastinal B-cell lymphoma, Burkitt lymphoma, lymphoplasmacytic lymphoma (i.e. Fahrenheit macroglobulinemia), hairy cell leukemia (HCL), immunoblastic large cell lymphoma, precursor B lymphoblastic lymphoma, and primary central nervous system (CNS) lymphoma; and T-cell NHL, such as precursor T-cell lymphoma/leukemia, peripheral T-cell lymphoma (PTCL) (such as cutaneous T-cell lymphoma (CTCL) (such as mycosisfungoides, Sezary syndrome), vascular immunobast T-cell lymphoma, extranodal natural killer T-cell lymphoma, intestinal type T-cell lymphoma, subcutaneous panniculitis-like T-cell lymphoma, and anaplastic large cell lymphoma); a mixture of one or more leukemia/lymphoma as described above; and multiple myeloma (MM) and heavy chain diseases (such as alpha chain disease, gamma chain disease, and µ chain disease); hemangioblastoma; hypopharyngeal cancer; inflammatory myofibroblastic tumor; immunocytic amyloidosis; renal cancer (such as nephroblastoma, also known as nephroblastoma or renal cell carcinoma); liver cancer (e.g. hepatocellular carcinoma (HCC), malignant hepatocellular carcinoma); lung cancer (such as bronchial cancer, small cell lung cancer (SCLC), non-small cell lung cancer (NSCLC), lung adenocarcinoma); smooth muscle sarcoma (LMS); mastocytosis (such as systemic mastocytosis); muscle cancer; myelodysplastic syndrome (MDS); mesothelioma; myeloproliferative disease (MPD) (such as polycythemia vera (PV), primary thrombocytosis (ET), agnogenic myeloid metaplasia (AMM), also known as myelofibrosis (MF), chronic idiopathic myelofibrosis, chronic myelocytic leukemia (CML), chronic neutropenic leukemia (CNL), hypereosinophilic syndrome (HES)); neuroblastoma; neurofibroma (e.g. type 1 or type 2 neurofibromatosis (NF), schwannoma); neuroendocrine carcinoma (e.g. gastrointestinal pancreatic neuroendocrine tumor (GEP-NET), carcinoid tumor); osteosarcoma (e.g. bone cancer); ovarian cancer (e.g. cystadenocarcinoma, ovarian embryonal carcinoma, ovarian adenocarcinoma); papillary adenocarcinoma; pancreatic cancer (such as pancreatic cancer, intraductal papillary mucinous tumor (IPMN), islet cell tumor); penile cancer (such as Paget's disease of penis and scrotum); pineal gland; primitive neuroectodermal tumor (PNT); plasma cell tumor; paraneoplastic syndrome; intraepithelial tumor; prostate cancer (such as prostate cancer); rectal cancer; rhabdomyosarcoma; salivary adenocarcinoma; skin cancer (such as squamous cell carcinoma (SCC), keratoacanthoma (KA), melanoma, basal cell carcinoma (BCC)); small intestine cancer (such as appendiceal cancer); soft tissue sarcoma (e.g. malignant fibrous histiocytoma (MFH), liposarcoma, malignant peripheral nerve sheath tumor (MPNST), chondrosarcoma, fibrosarcoma, myxosarcoma); sebaceous gland carcinoma; small intestine cancer; sweat gland carcinoma; synovioma; testicular cancer (e.g. seminoma, embryonal carcinoma of testis); thyroid cancer (e.g. papillary thyroid carcinoma, papillary thyroid carcinoma (PTC), medullary thyroid carcinoma); urethral cancer; vaginal cancer; and vulvar cancer (such as vulvar Paget disease).

### The fourth aspect of the present invention provides a pharmaceutical composition

A pharmaceutical composition is usually composed of main ingredient(s) and auxiliary ingredient(s), the pharmaceutical composition can be used for prevention or treatment, can be a standardized treatment or a personalized treatment (cell therapy, gene therapy).

The selection of main ingredients mainly includes nucleic acid elements, recombinant vectors, recombinant cells, etc. mentioned in the first and second aspects of the present invention as the main ingredients. Recombinant vectors can be formed by means of tandem connection, or by separately constructing vectors to form a mixture of multiple individual transcription factors. Preferably, mixing ratio is equal proportion mixing, and of course, different ratios are also allowed based on that the same active function is maintained.

The present invention further provides a use of the above-mentioned nucleic acid elements, recombinant vectors, recombinant cells, etc. in the preparation of medicament(s) for the treatment of disease(s) that can be prevented and treated by mediating cell rejuvenation. And a pharmaceutical composition for treating disease(s) that can be prevented and treated by mediating cell rejuvenation is provided. And a pharmaceutical composition is provided for the treatment of a disease that can be prevented and treated by mediating cell rejuvenation.

When the pharmaceutical composition is a combination of cells, especially a rejuvenated cell composition, it may also contain one or more additional factors, such as nutrients, cytokines, growth factors, extracellular matrix (ECM) components, antibiotics, antioxidants, or immunosuppressants, to improve cell function or survival ability.

Examples of growth factors include but are not limited to fibroblast growth factor (FGF), insulin-like growth factor (IGF), transforming growth factor-β (TGF-β), epiregulin, epidermal growth factor ("EGF"), endothelial growth factor ("ECGF"), nerve growth factor ("NGF"), leukemia inhibitory factor ("LIF"), bone morphogenetic protein 4 ("BMP-4"), hepatocyte growth factor ("HGF"), vascular endothelial growth factor-A ("VEGF-A"), and cholecystokinin octapeptide.

On the basis of the main ingredients, the pharmaceutical composition may optionally further include one or more pharmaceutically acceptable excipients, carriers, excipients, and vehicles. Examples of excipients, carriers, excipients, and vehicles include but are not limited to anti-adhesive agents, adhesives, coatings, compression aids, disintegrants, dyes, lubricants, emulsifiers, fillers (diluents), film-forming agents or coatings, flavorings, fragrances, glidants (flow enhancers), lubricants, adsorbents, suspension or dispersants, or sweeteners. Exemplary excipients, carriers, excipients, and vehicles include but are not limited to: butylated hydroxyl toluene (BHT), calcium carbonate, calcium phosphate (monohydro), calcium stearate, cross-linked carboxymethyl cellulose, cross-linked polyvinylpyrrolidone, citric acid, cross-linked polyvinylpyrrolidone, cysteine, ethyl cellulose, gelatin, hydroxypropyl cellulose, hydroxypropyl methyl cellulose, lactose, magnesium stearate, maltitol, mannitol, methionine, methyl cellulose, methyl p-hydroxybenzoate, microcrystalline cellulose, polyethylene glycol, polyvinylpyrrolidone, pre-gelatinized starch, propyl p-hydroxybenzoate, retinol palmitate, shellac, silica, carboxymethyl cellulose sodium, sodium citrate, sodium glycolate starch, sorbitol, starch (corn), stearic acid, stearic acid, sucrose, talc, titanium dioxide, vitamin A, vitamin E, Vitamin C and xylitol.

The pharmaceutical composition can be prepared in the form of injection formulations, oral formulations, inhalation formulations, or microneedle formulations. The injection formulations are: classified according to their physical state, including liquid injections, powders for injection, and tablets for injection; classified according to injection positions, including intradermal injection, subcutaneous injection, intramuscular injection, intravenous injection, intraperitoneal injection, and spinal cavity injection; preferably, the solvent for the injection formulation includes injection water or physiological saline. Intravenous, intramuscular, and microneedle injection methods are preferred.

Formulations for oral use include tablets containing active ingredients mixed with non-toxic pharmaceutically acceptable excipients. These excipients can be, for example, inert diluents or fillers (such as sucrose, sorbitol, sugar, mannitol, microcrystalline cellulose, starch including potato starch, calcium carbonate, sodium chloride, lactose, calcium phosphate, calcium sulfate or sodium phosphate); granulating agents and disintegrants (such as cellulose derivatives including microcrystalline cellulose, starch including potato starch, cross-linked carboxymethyl cellulose sodium, alginate or alginic acid); adhesives (such as sucrose, glucose, sorbitol, arabic gum, alginic acid, sodium alginate, gelatin, starch, pre-gelatinized starch, microcrystalline cellulose, magnesium aluminum silicate, sodium carboxymethyl cellulose, methyl cellulose, hydroxypropyl methyl cellulose, ethyl cellulose, polyvinyl pyrrolidone or polyethylene glycol); and lubricants, glidants, and anti-adhesive agents (such as magnesium stearate, zinc stearate, stearic acid, silica, hydrogenated vegetable oil, or talc). Formulations for oral use can also be in the form of chewable tablets or hard gelatin capsules, wherein the active ingredient is mixed with an inert solid diluent (such as potato starch, lactose, microcrystalline cellulose, calcium carbonate, calcium phosphate, or kaolin), or in the form of soft gelatin capsules, wherein the active ingredient is mixed with water or oil medium (such as peanut oil, liquid paraffin, or olive oil). Powders, granules and pills may be prepared in a conventional manner using, for example, a mixer, a fluidized bed device or a spray drying device using the ingredients mentioned above under tablets or capsules.

Other pharmaceutically acceptable excipients for oral formulations include but are not limited to colorants, flavorings, plasticizers, moisturizers, and buffering agents. Formulations for oral use can also be in the form of chewable tablets or hard gelatin capsules, wherein the active ingredient is mixed with an inert solid diluent (such as potato starch, lactose, microcrystalline cellulose, calcium carbonate, calcium phosphate, or kaolin), or in the form of soft gelatin capsules, wherein the active ingredient is mixed with water or oil medium (such as peanut oil, liquid paraffin, or olive oil). Powders, granules and pills may be prepared in a conventional manner using, for example, a mixer, a fluidized bed device or a spray drying device using the ingredients mentioned above under tablets or capsules.

The injection formulation includes injection solution, injection lotion, freeze-drying agent, suspension injection, etc. The excipients for injection formulations include injection water and at least one solubilizer. The solubilizer comprises at least one of tween 80, ethanol, propylene glycol, and polyethylene glycol 400. Preferably, the solubilizer is tween 80 or polyethylene glycol 400. Injection formulations also include but are not limited to protein nanoparticles, cyclodextrin inclusions, liposomes, microspheres, depot controlled release injections, or needle free injection drug release systems.

### Combination therapy

The nucleic acid elements, recombinant vectors, recombinant cells, and pharmaceutical compositions containing thereof involved in the present invention can be combined with other drugs for combination therapy. Taking anti-aging treatment as an example, initiation phase reprogramming can also be combined with protein therapy, such as B 18R protein, for anti-aging treatment.

### Beneficial effect

Cell rejuvenation occurs in the early-stage of reprogramming, while loss of somatic identity only occurs in the middle- and late- stage of reprogramming. Therefore, there is a time window here to rejuvenate cells while maintaining their original cell identity. This is the technical principle of partial reprogramming to rejuvenate cells. The inventor found that after the transcription factors lose their phase separation ability, it is difficult for the reprogramming to enter the maturation phase, resulting in retantion in the initiation phase without loss of cell identity. Therefore, it increases the degree of cell rejuvenation while reducing the risk of iPSC generation, and does not completely reprogram, hence it is called "initiation phase reprogramming". Validation of cell reprogramming phase is ensured by judgment with the phenotype of functional genes, it has been proven that cells have achieved the technological effect of rejuvenation.

In clinical practice, as cells age, functions of cell itself gradually decrease, leading to aging and disorder at the tissue, organ, and systemic levels. A large number of age-related diseases, including but not limited to neurodegenerative diseases, cardiovascular and peripheral vascular diseases, eye diseases, skin diseases, autoimmune diseases, metabolic dysfunction, musculoskeletal diseases, digestive system diseases, cell proliferation related conditions, and tumors, are fundamentally caused by the aging and dysfunction of related tissues and organs. Through cell rejuvenation therapy for specific tissues and organs, it can effectively alleviate and treat related diseases, benefiting the general public. At the same time, extending healthy lifespan is beneficial for greatly improving social productivity, alleviating medical needs and pressures, and creating new wealth for an aging society.

### Figures

Figure 1: Construction of lentiviral vector. (A) Schematic diagram of lentiviral vector structure; (B) Identification of lentiviral vector plasmid by enzyme cleavage.
Figure 2: Lentiviral Quality inspection. (A) Results of lentivirus titer test and sterility test; (B) The expression level of transgenic KLF4 after lentiviral transduction of 293T cells.
Figure 3 is a schematic diagram of the structure of the lentiviral vector used in Example 2.
Figure 4: Senescence-associated β-galactosidase (SA-β-Gal) staining experiment. A) Representative image of staining results for the blank control group. B) Representative image of staining results of OKMS reprogramming group. C) Representative image of staining results of the O'KM'S reprogramming group. D) Statistics of the proportion of SA-β-Gal staining positive cells in each group.
Figure 5: Detection of expression levels of genes related to the rejuvenation function of skin fibroblasts. The significance of the difference between the OKMS reprogramming group and the O'KM'S reprogramming group was confirmed by t-test. **: P < 0.01, ***: P < 0.001.
Figure 6: Detection of gene expression levels of skin fibroblast identity markers. The significance of the difference between the OKMS reprogramming group and the O'KM'S reprogramming group was confirmed by t-test. *: P < 0.05, **: P < 0.01, ***: P < 0.001.
Figure 7: Detection of gene expression levels of the cell reprogramming initiation phase markers. The significance of the difference between the OKMS reprogramming group and the O'KM'S reprogramming group was confirmed by t-test. **: P < 0.01.
Figure 8: Detection of gene expression levels of the cell reprogramming maturation phase markers. The significance of the difference between the OKMS reprogramming group and the O'KM'S reprogramming group was confirmed by t-test. **: P < 0.01, ***: P < 0.001.
Figure 9: Results of epigenetic age (epiAge) analysis of cellular DNA methylation.
Figure 10: Structural diagram of the lentiviral vector used in Example 3
Figure 11: Senescence-associated β-galactosidase (SA-β-gal) staining experiment. A) Representative image of staining results for the blank control group. B) Representative image of staining results of OKM reprogramming group. C) Representative image of the staining results of the O'KM' reprogramming group. D) Statistics of the proportion of positive staining cells in each group.
Figure 12: Detection of expression levels of genes related to rejuvenation function of skin fibroblasts. The significance of the difference between the OKM reprogramming group and the O'KM' reprogramming group was confirmed by t-test. **: P < 0.01, ***: P < 0.001.
Figure 13: Detection of gene expression levels of skin fibroblast identity markers. The significance of the difference between the OKM reprogramming group and the O'KM' reprogramming group was confirmed by t-test. ***:
Figure 14: Detection of gene expression levels of the cell reprogramming initiation phase markers. The significance of the difference between the OKM reprogramming group and the O'KM' reprogramming group was confirmed by t-test. n.s.: not significant, * * *: P<0.001.
Figure 15: Detection of gene expression levels of the cell reprogramming maturation phase markers. The significance of the difference between the OKM reprogramming group and the O'KM' reprogramming group was confirmed by t-test. ***:P < 0.001.
Figure 16: AAV vector construction. (A) Schematic diagram of AAV-TRE3G-OKS vector structure; (B) Identification of AAV-TRE3G-OKS vector plasmid by enzyme cleavage; (C) Schematic diagram of structure of AAV-TRE3G-O'KS vector; (D) Identification of AAV-TRE3G-O'KS vector plasmid by enzyme cleavage.
Figure 17: Determination of AAV vector titers by qPCR method. (A) Original amplification curve group; (B) Standard curve; (C) Determination results of AAV vector titer.
Figure 18 shows construction of circular RNA vectors with different combinations of partial reprogramming factors.
Figure 19 shows validation of circular RNA ring formation using different combinations of partial reprogramming factors. (A) The results of 2% E-gel electrophoresis showed that MYC-circRNA, KLF4-circRNA, OCT4-P2A-SOX2-circRNA, and Lin28A-P2A-Nanog-circRNA had circular RNA formation, as indicated by the arrows. The results of (B and C) 0.6% agarose gel electrophoresis for 30 minutes (top) and 90 minutes (bottom) showed that OSK circRNA, O'SK-circRNA and O'KM'-circRNA, OKM-circRNA, OKMS-circRNA had circular RNA formation, as indicated by the arrows.
Figure 20: Validation results of protein expression WB from HEK293T cells transfected with circular RNA of partial reprogramming factor.
Figure 21: Wild-type OKSMLN factor circRNA was transfected into fibroblasts to induce iPSC, and clones appeared on day 7.

### Detailed Description

The present invention will be further explained in conjunction with specific examples. It should be understood that these examples are only used to illustrate the present invention and not to limit the scope of the present invention. The experimental methods in the following examples that do not specify specific conditions are usually carried out under conventional conditions such as those described in J. Sambrook et al., Molecular Cloning Experiment Guide, Third Edition, Science Press, 2002, or as recommended by the manufacturer.

### Example 1: Gene synthesis and construction of lentivirus (LV) vector

### 1. Sequence design of single element of transcription factors

Design the sequences of OCT4' and variants OCT4'1-6, as shown in SEQ ID NOs: 4-10, and design the corresponding nucleotide sequences, for example, as shown in SEQ ID NO: 3; design the sequences of C-MYC and its variants, as shown in SEQ ID NOs: 14-20, and design the corresponding nucleotide sequences, for example, as shown in SEQ ID NO: 13; design the KLF4 sequence, as shown in SEQ ID NO: 22, and design the corresponding nucleotide sequence, as shown in SEQ ID NO: 21; design the SOX2 sequence, as shown in SEQ ID NO: 24, and design the corresponding nucleotide sequence, for example, as shown in SEQ ID NO: 23.

Design linker sequences, P2A, T2A, E2A sequences, for example, as shown in SEQ ID NOs: 25-27; design a Kozak sequence, GCCGCCACC; design a TRE3G promoter sequence, as shown in SEQ ID NO: 63; design the SV40 poly (A) sequence as shown in SEQ ID NO: 64.

### 2. Construction of a sequence of transcription factor elements in tandem

The sequences connected in tandem by the aforementioned elements are provided solely as examples, may be those shown in SEQ ID NOs: 28-33, named OCT4'-P2A-KLF4s-T2A-MYC'-E2A-SOX2 tandem sequence (abbreviated as O'KM'S, corresponding to SEQ ID NO: 28), OCT4-P2A-KLF4s-T2A-MYC-E2A-SOX2 tandem sequence (abbreviated as OKMS, corresponding to SEQ ID NO: 29), OCT4'-P2A-KLF4s-T2A-MYC' tandem sequence (abbreviated as O'KM', corresponding to SEQ ID NO: 30), OCT4-P2A-KLF4s-T2A-MYC tandem sequence (abbreviated as OKM, corresponding to SEQ ID NO: 31), OCT4'-P2A-KLF4s-T2A-SOX2 tandem sequence (abbreviated as O'KS, corresponding to SEQ ID NO: 32), and OCT4-P2A-KLF4s-T2A-SOX2 tandem sequence (abbreviated as OKS, corresponding to SEQ ID NO: 33), respectively.

### 3. Construction and packaging of lentiviral vectors (carried out by Cyagen Bioscience, Inc.)

### 3.1 Construction of lentiviral vectors:

### 1) Gene synthesis

Gene synthesis of O'KM'S, OKMS, O'KM' and OKM fragments.

### 2) Enzyme cleavage of vector backbone

Appropriate enzyme cleavage sites on a vector backbone are selected for enzyme cleavage.

The CutSmart system from NEB used for enzyme cleavage is as follows

| | Materials | | System |
|---|---|---|---|
| | CutSmart | | 17.5 µL |
| | Plasmid | | 600 ng~800 ng |
| | Restriction endonuclease | | 0.7 µL or 2×0.5 µL |

After enzyme cleavage, the gel recovery kit (NEB Monarch DNA) is used to recover the bands. Prepare the required backbone fragments and target fragments for subsequent ligation experiments.

### 3) Ligation and transformation

The synthesized genes and other related elements are linked to the linearized vector backbone in sequence, respectively. The C115 Infusion ligase system is used for ligation, as follows:

| | Added materials | | Volume |
|---|---|---|---|
| | Treated backbone | | 100 ng |
| | Ligaion fragment | | 50 ng |
| | 2×enzyme mixture | | 5 µL |
| | Sterilized water | | Up to 10 µL |

| | C115 Infusion enzyme ligation reaction procedure | | |
|---|---|---|---|
| | Reaction temperature | | 50 °C |
| | Hot cover temperature | | 105 °C |
| | Reaction time | | 15 min |
| | Reaction system | | 10 µL |

After the ligation reaction was completed, the corresponding antibiotic plate was transferred to a 37 °C incubator to invert and dry while 500µL non-antibiotic medium was pre-warmed; the competent cells were taken out of the -80 °C freezer, keep them on ice for 15-20 minutes until thawed. A half of the ligation solution was pipetted into the competent cells, with the tube flicked gently for five times, followed by incubation on ice for 30 minutes; the competent cells was then rapidly transferred to preheated 42°C water bath for heat-shock of 1 minute (heat-shock time of 45-90s, strictly timed) and immediately transferred and placed on ice for 2 minute. Cells were transferred into the pre-warmed 500 µL medium and shaken at 37°C for 1 h; bacterial suspension was taken and centrifuged at 4000 rpm for 2 min; and 400 µL supernatant was discarded. About twenty glass beads were added to the plate, the remaining cell suspension was mixed well by pipetting, spreaded on the corresponding antibiotic plate, and incubated overnight in the corresponding incubator.

After overnight incubation, bacterial test is performed: turn on the UV lamp in the clean bench for 20 min, then switch it off and set the blower to level 3; wipe the work surface with 75 % ethanol and proceed with bacterial test; add 25 µL sterilized water to the bottom of the 96-well PCR plate on the clean bench, and check for air at the bottom under light; gently dip the sterilized 10 µL white pipette tip into the single clone bacterial plaque on the overnight incubated plate, pick it up into the corresponding 96-well reaction plate, and use a 10 µL pipette to blow and mix 2-3 times. Draw 4 µL each row and transfer it to a new 96-well reaction plate as a template for PCR reaction. Add 50 µL of non-antibiotic broth culture medium into the original 96-well plate template, cover with sterilized 96-well PCR plate cover, and place in a 30°C incubator.

### 4) Bacterial test

### P222-AA Taq enzyme reaction system was used for bacterial test as follows

| | Added materials | | System |
|---|---|---|---|
| | 2×Taq enzyme mixture | | 12.5 µL |
| | Single colony water suspension | | 4 µL |
| | 2×enzyme mixture | | 2×0.75 µL |
| | Sterilized water | | Up to 25 µL |

### P222-AA Taq enzyme reaction procedure

| | Temperature | | | Time | Cycles |
|---|---|---|---|---|---|
| Pre denaturation | 95 °C | | | 3 min | |
| Denaturation | 95 °C | | | 15 s | 23 |
| Annealing | 60°C | | | 15 s | |
| Extension | 72°C | | | 15 s/kb | |
| Final extension | 72°C | | | 5 min | |
| Store | 25°C | | | +∞ | |

After the bacterial test reaction is completed, perform TBE gel detection to check if the band size matches the theoretical band size. Then, transfer 3.5 mL of Amp resistant broth medium into a 48 deep well plate and place it overnight on a 37°Cshaker for culture.

### 5) Plasmid extraction

Plasmid extraction was performed using QIAprep Spin Miniprep Kit (27106) from Qiagen. The extracted plasmids were subjected to concentration determination and labeled the concentration on the wall of a 1.5mL EP tube. A concentration greater than 50ng/µ L was considered successful extraction and could be subjected to enzyme cleavage identification.

### 6) Enzyme cleavage identification

NTI/SnapGene software was used to search for available enzyme cleavage protocol based on existing endonucleases, and to identify whether the plasmid is correct. The enzyme cleavage system is as follows

| | Added materials | | System |
|---|---|---|---|
| | Buffer | | 2 µL |
| | Single enzyme/dual enzymes | | 0.7 µL/0.5 µL |
| | Plasmid | | 600~800 ng |
| | Sterilized water | | Up to 20 µL |

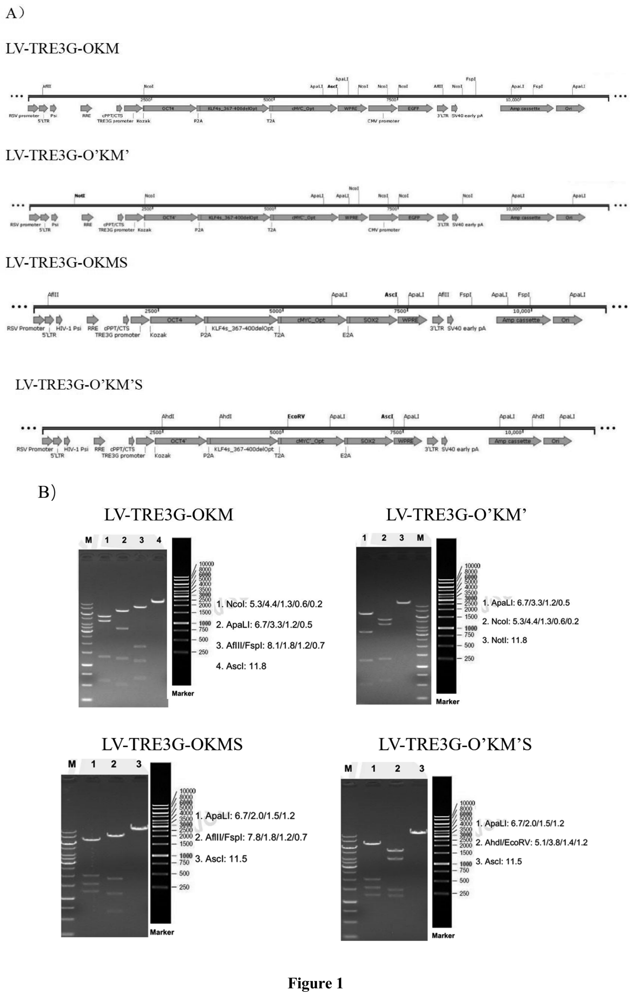

After preparing the system, place it at the appropriate temperature of the corresponding enzyme for 45min, take out the enzyme-cleaved system, add 5ul bromophenol blue/methyl orange to perform TAE gel detection, and then take photos with the gel imager to check the size of bands (see Figure 1 for the results).

If the bands are correct, draw 30 µL of plasmid on the same day and prepare for testing. Send to GENEWIZ to sequence the sequence of ligation area.

### 7) Plasmid sequencing

The constructed plasmid was sent to GENEWIZ for sequencing, which confirmed the successful construction of the vector.

### Materials

| | Materials | | Source |
|---|---|---|---|
| | PCR enzyme | | Vazyme P515 |
| | PCR gel recovery kit | | QIA Gel Extraction Kit(28706) |
| | Ligase | | Vazyme C115 |
| | Bacterial test Taq enzyme | | Vazyme P222 |
| | Competent cells | | WEIDI DH5α |
| | Plasmid extraction | QIAprep Spin Miniprep Kit(27106) | |
| PCR, primer synthesis and gene synthesis for bacterial test and sequencing | | | GENEWIZ |

Successfully constructed lentiviral vectors, including LV-TRE3G-OKM, LV-TRE3G-O'KM', LV-TRE3G-OKMS, LV-TRE3G-O'KM'S, plasmid sequences of which are as shown in SEQ ID NOs: 34-37.

### 3.2 lentivirus packaging:

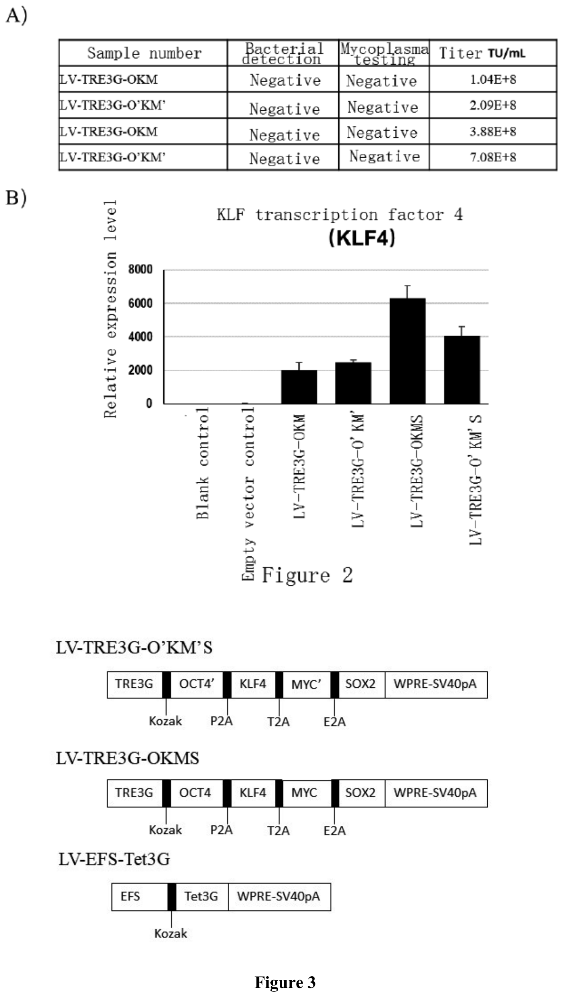

1) Lentiviral plasmids and helper plasmids (SL3, SL4, and SL5) co infect into 293T cells.
2) Virus harvesting: after transfection for 48 hours, collect the cell supernatant by centrifugation.
3) Conversion concentration: PEG8000 concentration and sucrose density gradient ultracentrifugation were performed on the above-mentioned supernatant of the cells.
4) Quality inspection: physical state testing, sterility testing, and titer testing, etc. The titer testing uses qPCR method (see Figure 2A for results).
5) Transgenic expression detection: the above lentiviral vectors were co-transduced into 293T cells together with a TET3G-expressing lentiviral vector, and expression was induced by adding doxycycline (dox). RNA sample was extracted 48 h later, and expression level of transgenic KLF4 was detected using the KLF4-specific primers (results shown in Figure 2B).

### Example 2: Four factor initiation phase reprogramming rejuvenates human dermal fibroblasts

### Experimental protocol:

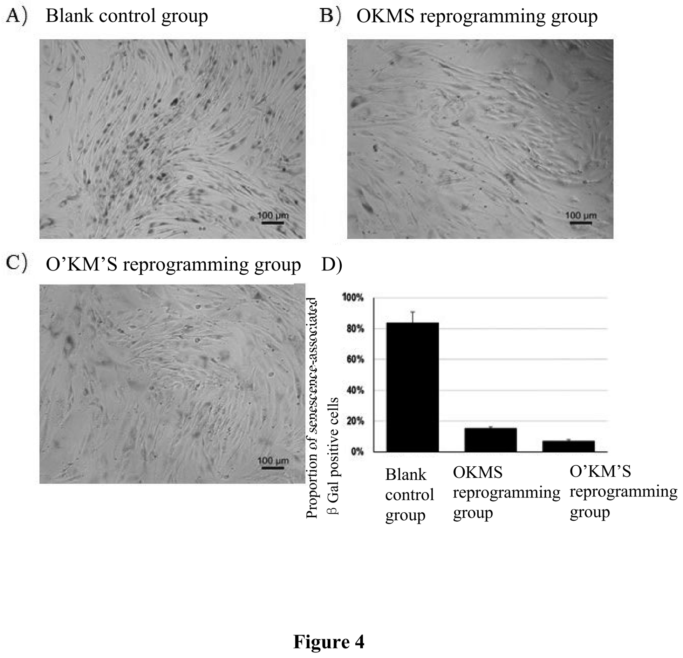

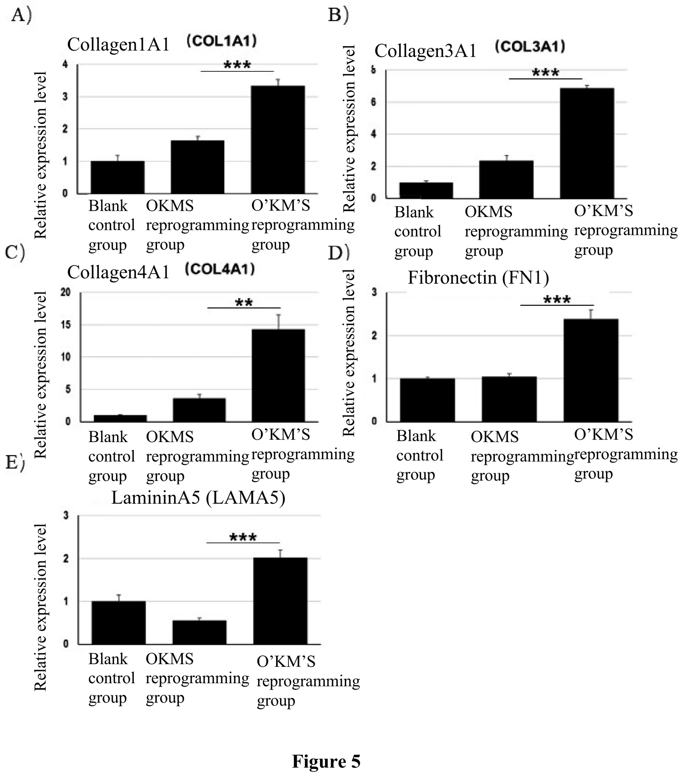

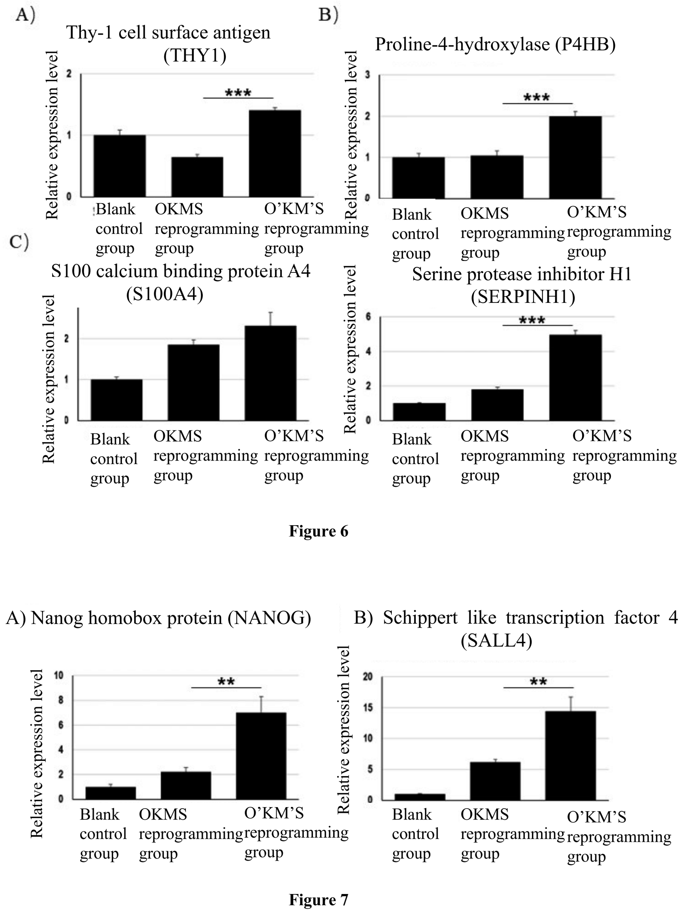

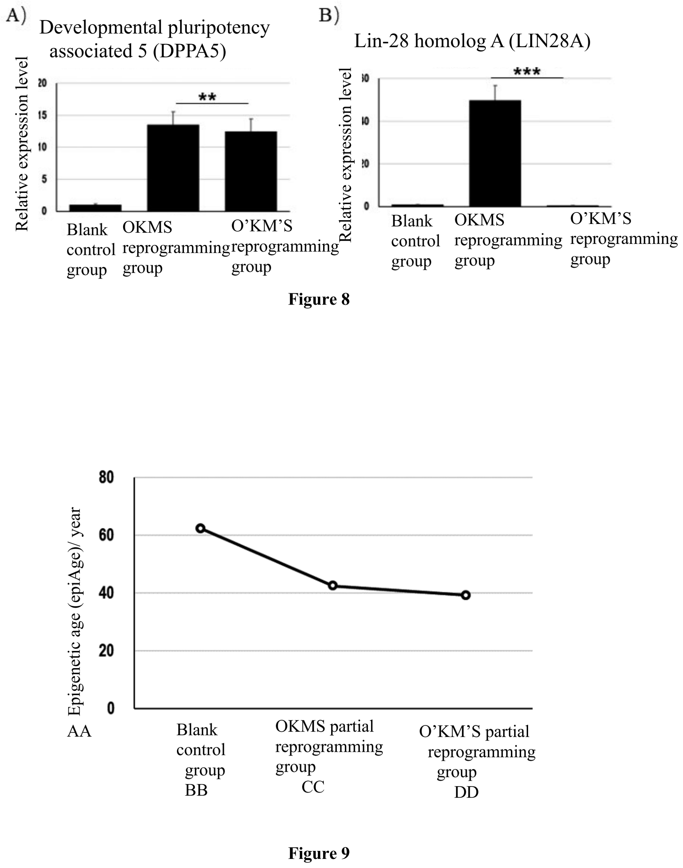

1) Select a primary human skin fibroblast cell line FY-009 from a female donor over 60 years old, culture it in a specialized epidermal cell culture medium, passage, and select a cell line in good condition for subsequent viral infection experiments;
2) After the fourth generation of in vitro culture of fibroblasts, they are seeded onto a 12 well plate and divided into three groups with a confluence of about 40%: the first group was a blank control group without virus addition; the second group of OKMS reprogramming, adding lenti virus LV-TRE3G-OKMS and LV-EFS-Tet3G; the third group of O'KM'S reprogramming, adding lenti virus LV-TRE3G-O'KM'S and LV-EFS-Tet3G. The lentiviral vector was prepared by Cyagen Bioscience Inc., and the structural diagram is shown in Figure 3. Polybrene was simultaneously added to enhance the efficiency of viral infection.
3) After 24 hours, discard the supernatant and replace it with a high glucose DMEM culture medium containing 20% fetal bovine serum.
4) Continue to change the medium, add doxycycline (dox) to induce transcription factor expression, and change the medium every two days thereafter.
5) After inducing with dox for 14 days, collect cells from each group for detection.
6) Perform senescence-associated β-galactosidase (SA-β-gal) staining on each group of cells to detect their degree of aging (experimental results are shown in Figure 4). The results showed that both reprogramming groups were able to rejuvenate cells, with the O'KM'S reprogramming group showing a higher degree of rejuvenation.
7) Extract RNA samples from each group of cells and perform real-time fluorescence quantitative polymerase chain reaction (q-PCR) to detect genes related to rejuvenation function: COL1A1, COL3A1, COL4A1, FN1, LAMA5 ( see Figure 5 for experimental results); detect fibroblast identity feature genes: THY1, P4HB, S100A4, SERPINH1 (see Figure 6 for experimental results); detect feature genes of the initiation phase of reprogramming: NANOG, SALL4 (see Figure 7 for experimental results); detect feature genes of maturation phase of reprogramming: DPPA5, LIN28A (see Figure 8 for experimental results). The results showed that the O'KM'S reprogramming group had significantly higher expression of rejuvenation function related genes and identity feature genes in cells compared to the OKMS reprogramming group. Moreover, from the perspective of feature genes of the reprogramming phase, the former was still in the initiation phase, while the latter had entered the maturation phase.
8) Collect cells from each group for methylation chip detection, and perform epiAge analysis on the detection results (results are shown in Figure 9). The results showed that the epiAge of the blank control group was over 60 years old, which was close to the actual age of the donor. The partial reprogramming group of OKMS reduced the cell's epiAge to 42 years, while the partial reprogramming group of O'KM'S reduced the cell's epiAge even further to 39 years.

### Example 3: 3-factor initiation phase reprogramming rejuvenates human skin fibroblasts

### Experimental protocol:

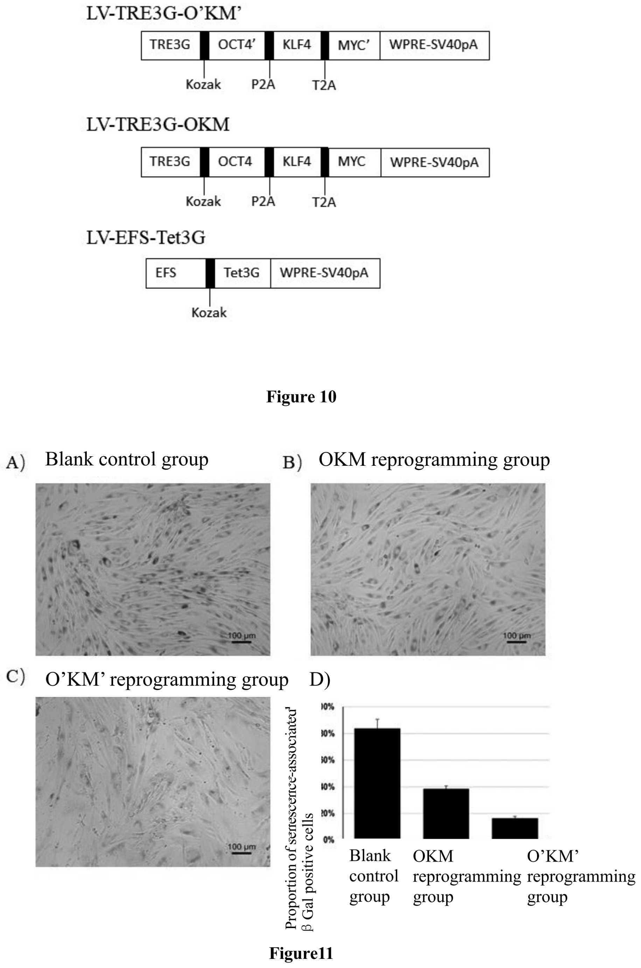

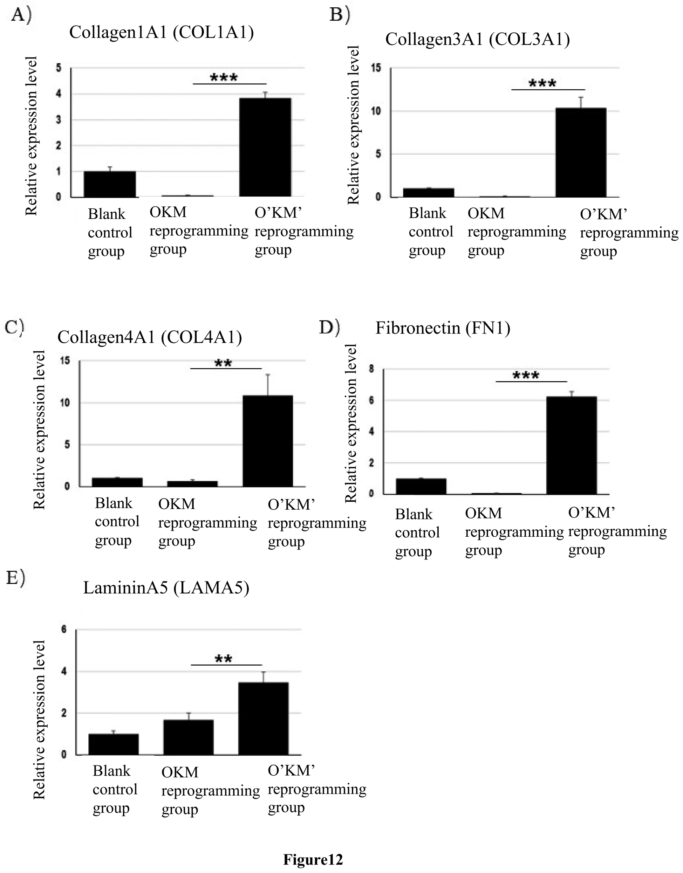

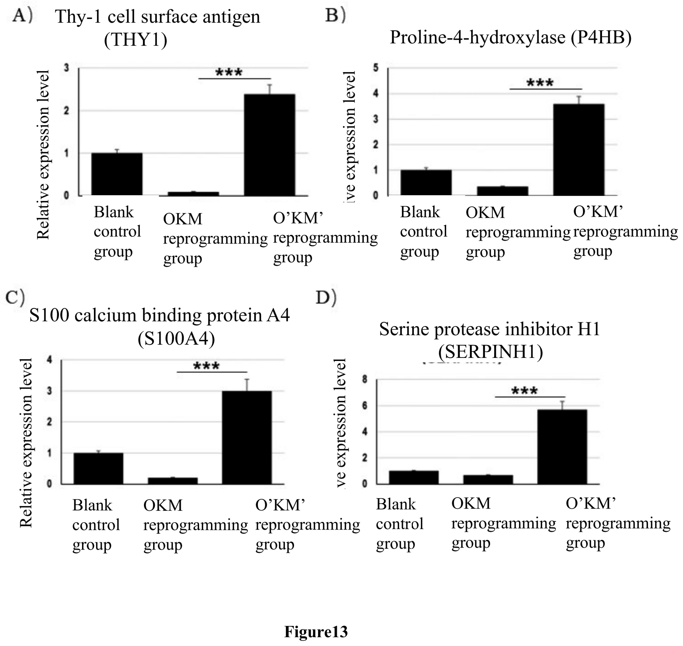

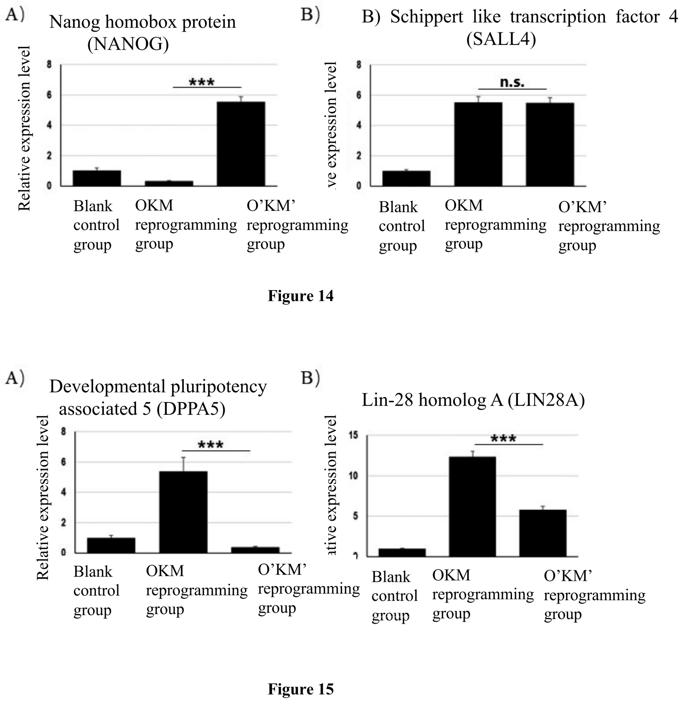

1) Human skin fibroblasts are cultured in specialized epidermal cell culture medium, passaged, and cell lines in good condition are selected for subsequent viral infection experiments;
2) After the fourth generation of in vitro culture of fibroblasts, they were seeded onto a 12-well plate and divided into three groups with a confluence of about 40%: the first group was a blank control group without virus addition; the second group of OKM reprogramming, adding lenti viruses LV-TRE3G-OKM and LV-EFS-Tet3G; the third group of O'KM reprogramming, adding lenti virus LV-TRE3G-O'KM' and LV-EFS-Tet3G. The lentiviral vector was prepared by Cyagen Bioscience Inc., and the structural diagram is shown in Figure 10. Polybrene was simultaneously added to enhance the efficiency of viral infection.
3) After 24 hours, discard the supernatant and replace it with a high glucose DMEM culture medium containing 20% fetal bovine serum.
4) Continue to change the medium, add doxycycline (dox) to induce transcription factor expression, and change the medium every two days thereafter.
5) After inducing with dox for 14 days, collect cells from each group for detection.
6) Perform senescence-associated β-galactosidase (SA-β-gal) staining on each group of cells to detect their degree of aging (experimental results are shown in Figure 11). The results showed that both reprogramming groups were able to rejuvenate cells, but the degree of cell rejuvenation in the OKM reprogramming group was lower, while the degree of rejuvenation in the O'KM' reprogramming group was higher.
7) Extract RNA samples from each group of cells and perform real-time fluorescence quantitative polymerase chain reaction (q-PCR) to detect genes related to rejuvenation function: COL1A1, COL3A1, COL4A1, FN1, LAMA5 (experimental results are shown in Figure 12); Detect fibroblast identity feature genes: THY1, P4HB, S100A4, SERPINH1 (experimental results are shown in Figure 13); Detect feature genes of the initiation phase of reprogramming: NANOG, SALL4 (experimental results are shown in Figure 14); Detect feature genes of maturation phase of reprogramming: DPPA5, LIN28A (experimental results are shown in Figure 15). The results showed that the O'KM' reprogramming group had significantly higher expression of rejuvenation function related genes and identity feature genes in cells compared to the OKM reprogramming group. Moreover, from the perspective of feature genes of the reprogramming phase, the former was still in the initiation phase, while the latter had entered the maturation phase.

### Example 4 Gene synthesis and construction of adeno-associated virus (AAV) vector

### 1. Sequence design of transcription factor elements

Design the sequences of OCT4' and variants OCT4'1-6, as shown in SEQ ID NOs: 4-10, and design the corresponding nucleotide sequences, as shown in SEQ ID NO: 3; design the sequences of C-MYC and its variants, as shown in SEQ ID NOs: 14-20, and design the corresponding nucleotide sequences, as shown in SEQ ID NO: 13; design the KLF4 sequence, refer to SEQ ID NO: 22, and design the corresponding nucleotide sequence, as shown in SEQ ID NO: 21; design the SOX2 sequence, refer to SEQ ID NO: 24, and design the corresponding nucleotide sequence, as shown in SEQ ID NO: 23.

Design linker subsequences, P2A, T2A, E2A sequences, as shown in SEQ ID NOs: 25-27; design Kozak sequence, gccgccaccatgg; design the TRE3G promoter sequence as shown in SEQ ID NO: 63; design the SV40 poly (A) sequence as shown in SEQ ID NO: 65.

### 2. Constructing a sequence of transcription factor elements in tandem

The sequences connected in tandem by the aforementioned elements are provided, only as examples, and may be those shown in SEQ ID NOs: 32-33, named OCT4'-P2A-KLF4s-T2A-SOX2 tandem sequence (abbreviated as O'KS, corresponding to SEQ ID NO: 32), OCT4-P2A-KLF4s-T2A-SOX2 tandem sequence (abbreviated as OKS, corresponding to SEQ ID NO: 33), respectively.

### 3. Construction and packaging of AAV vectors (executed by Cyagen Biotechnology Co., Ltd.)

### 3.1 AAV Vector Construction:

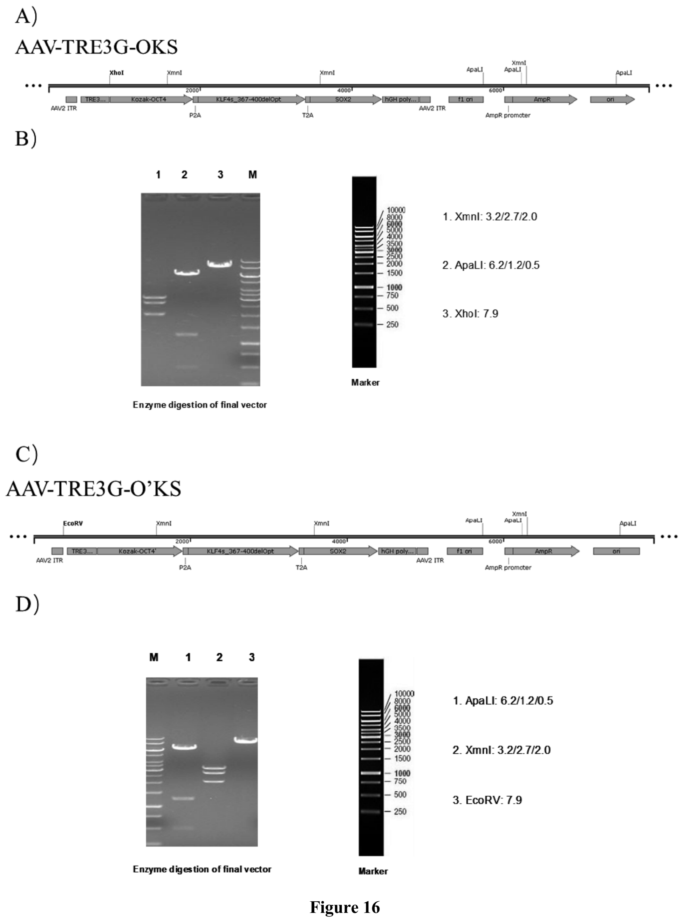

1) Select the appropriate combination of cleavage sites on the AAV skeleton plasmid, incubate the AAV skeleton plasmid and enzyme at 37°C for 1 hour, and then recover the corresponding linear skeleton plasmid after cleavage through agarose gel electrophoresis.
2) Gene synthesis of OKS and O'KS fragments, cloning of gene fragments and other related elements onto linear backbone plasmids using homologous recombination assay kit (Novozymes, C115), the reaction system was transformed into competent bacteria, coating plate, picking bacteria, shaking, and extracting. Perform enzyme cleavage and sequencing identification.
3) After confirming the accuracy of the enzyme cleavage and sequencing identification results (see Figure 16 for the enzyme cleavage identification results), select the correct plasmid from the bacterial solution and transform it into competent bacteria again. Coat, pick bacteria, amplify, and extract the plasmids required for AAV packaging, including AAV-TRE3G-OKS and AAV-TRE3GO'KS. The plasmid sequences are shown in SEQ ID NOs: 38-39.

### 3.2 AAV packaging:

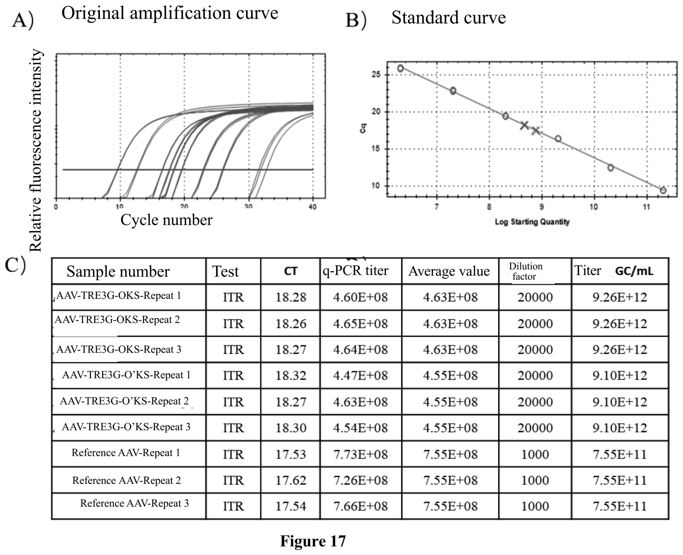

1) AAV virus plasmids, helper plasmids, and packaging plasmids co-infect 293T cells.
2) Virus harvesting: after transfection for 48 hours, collect the cell supernatant by centrifugation.
3) Conversion concentration: PEG8000 concentration and sucrose density gradient ultracentrifugation were performed on the supernatant of the above cells.
4) Quality inspection: physical state testing, sterility testing, and titer testing, etc. The titer testing uses qPCR method (see Figure 17 for titer testing results).

### Example 5: Gene synthesis and construction of circular mRNA vector

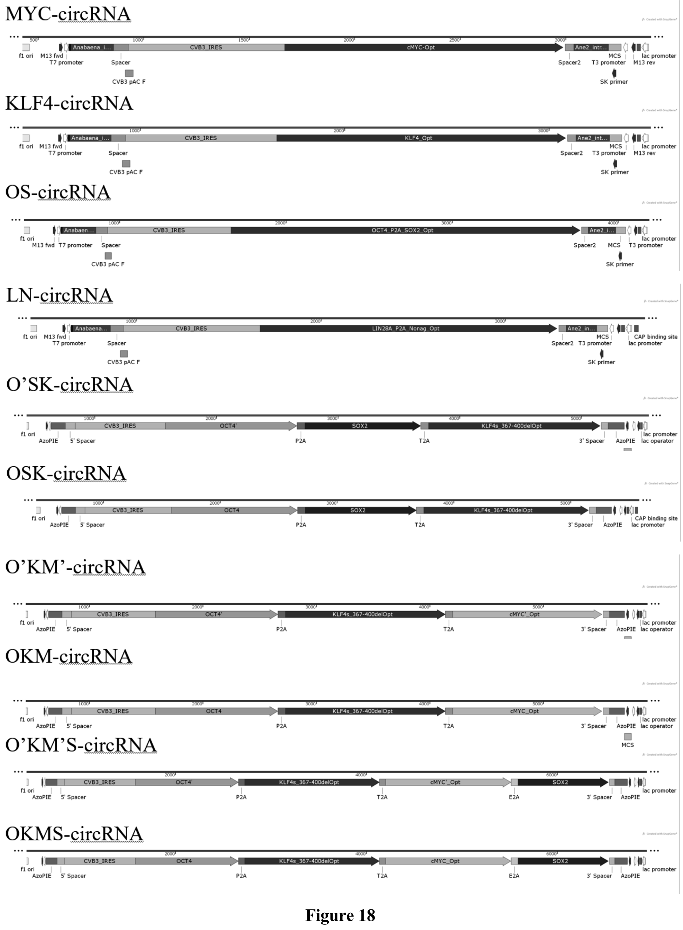

1. Construction of circular RNA vectors with different tandem combinations of partial reprogramming factors
a) Design of circular RNAs with different tandem combinations of partial reprogramming factors: concatemers of single gene, double genes, triple genes, and quadruple genes were designed, and different genes were in tandem with P2A, T2A, E2A tandem peptides. The schematic diagram of the vector structure for different gene tandem combinations is shown in Figure 18 and Table 1.

| Sequence | Gene combination | Sequence size(bp) | Circular RNA ring forming system |
|---|---|---|---|
| M | MYC | 1320 | AnaPIE |
| K | KLF4 | 1413 | AnaPIE |
| LN | Lin28A-P2A-Nanog | 1611 | AnaPIE |
| OS | OCT4-P2A-SOX2 | 2100 | AnaPIE |
| O'SK | OCT4-P2A-SOX2-T2A-KLF4 | 3573 | AzoPIE |
| OSK | OCT4'-P2A-SOX2-T2A-KLF4 | 3573 | AzoPIE |
| O'KM' | OCT4'-P2A-KLF4-T2A-MYC' | 3939 | AzoPIE |
| OKM | OCT4-P2A-KLF4-T2A-MYC | 3939 | AzoPIE |
| O'KM'S | OCT4'-P2A-KLF4-T2A-MYC'-E2A-SOX2 | 4959 | AzoPIE |
| OKMS | OCT4-P2A-KLF4-T2A-MYC-E2A-SOX2 | 4959 | AzoPIE |

b) Selection of ring forming elements: using Anabaena group I intron from the Genus Goldfish or group I intron from Azoarcus sp. BH7 of the Genus Azoarcus rearranged and formed PIE (permuted intron-exon) ring forming framework, different partial reprogramming factor combined with the target gene sequence were recombined into the PIE framework and recombined into pBlueScript II SK (+) plasmid
AnaPIE elements:
   5' element sequence is shown in SEQ ID NO: 49; 3' element sequence is shown in SEQ ID NO: 50.
AzoPIE elements:
   5' element sequence is shown in SEQ ID NO: 51; 3' element sequence is shown in SEQ ID NO: 52.
c) Plasmid synthesis: the recombinant plasmid is entrusted to a third-party sequence synthesis company for synthesis. The synthesized plasmid is linearized using EcoR I template to obtain a pre-transcriptional template.

2. Preparation and verification of circular RNAs with different tandem combinations of partial reprogramming factors
a) In vitro transcription and cyclization of circular RNA:
The in vitro transcription system is as follows: Table 2
Incubate at 37°C for 3 hours in a PCR machine, then digest the linear DNA template with DNase

| **In vitro transcription(20µL system)** | |
|---|---|
| **Components** | **Volume** |
| ATP/CTP/UTP/GTP(100mM) | each 2µL |
| 10×T7 buffer | 2µL |
| T7 RNA polymerase(50U/µL) | 2µL |
| RNAse inhibitor(40U/µL) | 1µL |
| Inorganic pyrophosphatase(0.1U/µL) | 2µL |
| Linear template DNA | 1µg |
| Enzyme free water | Up to 20µL |

I (5U/µL) at a concentration of 5U/µg DNA template, with the digestion condition of 37°C for 20 minutes.
Cyclization reaction:
Prepare the cyclization system, as shown in Table 3

| **Cyclization system(50µL system)** | |
|---|---|
| **Components** | **Volume** |
| GTP(100mM) | 1µL |
| 10×T7 enzyme buffer | 3µL |
| In vitro transcription products | 23µL |
| Enzyme free water | Up to 50µL |

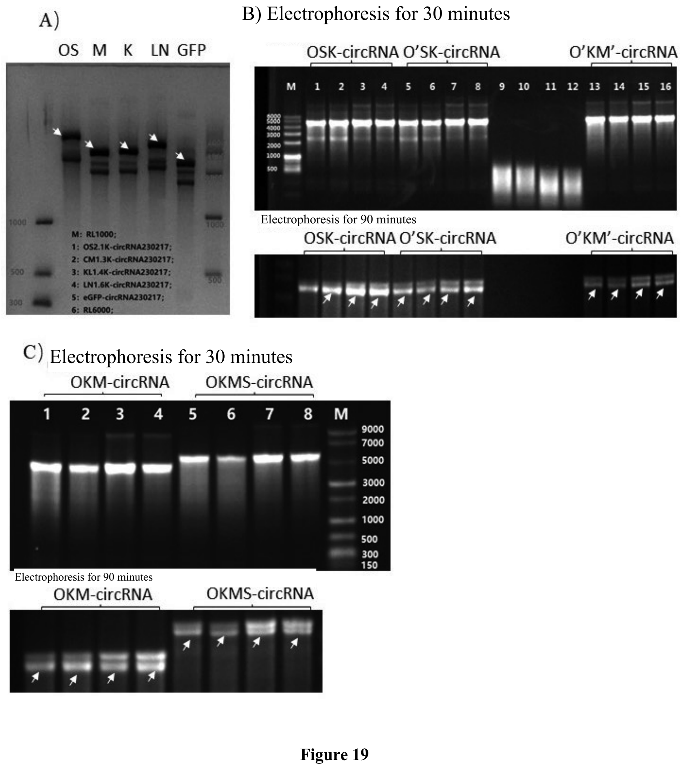

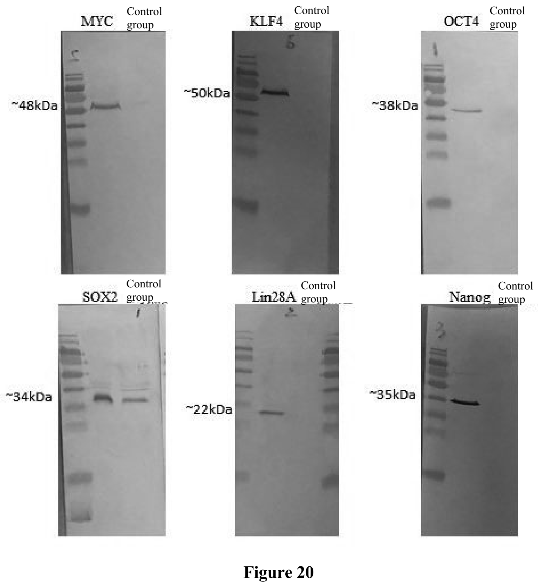

Place the prepared cyclization system in a PCR machine and incubate at 60°C for 20 minutes.
b) Purification and identification of circular RNA:
Transfer the cyclization system to a 1.5mL centrifuge tube, add 0.5 times the volume of a 7.5M lithium chloride and 50mM EDTA RNA precipitation solution, mix well and precipitate at -20°C for 1 hour. After precipitation is complete, centrifuge at 18360×g for 15 minutes at 4°C to remove the supernatant as much as possible. Add 800µL of 70% ethanol solution, rinse the precipitate, centrifuge again under the same conditions, try to remove the supernatant as much as possible, and then evaporate the ethanol. Each experimental group is resuspended in 100µL of enzyme free water. Determine the concentration value of the content and identify the size of circRNA by 1% agarose gel electrophoresis or 2% E-Gel electrophoresis. The electrophoresis results showed the formation of circular RNA, as shown in Figure 19.
3. Verification of protein expression in eukaryotic cells transfected with partial reprogramming factors
a) Cell preparation and mRNA transfection:
   Take a bottle of HEK293T/17 cells cultured in T75 and plate them at a cell confluence of 80% - 90%. Discard the culture medium, wash with PBS, discard PBS, and add 0.05% trypsin to digest the cells. Wait for the cells to become round, discard the trypsin, and blow the cells with 10mL of antibiotic free 10% FBS cell culture medium to disperse them. Cell counting, adjust the cell concentration to 1×10⁶ cells/mL±10%, take 10 48-well plates, inoculate 0.2mL of cell suspension separately, and place the cell culture plates in a 37 °C, 5% CO₂ incubator for 24 hours±4 hours.
   Dissolve MYC-circRNA, KLF4-circRNA, OCT4-P2A-SOX2-circRNA, and Lin28A-P2A-Nanog-circRNA each at 0.4ug circRNA in 25 µL serum-free DMEM medium, gently mix well, and let stand at room temperature for 5 minutes. Dissolve 1 µL Lipohigh transfection reagent in 25 µL serum-free DMEM medium, gently mix well, and let it stand at room temperature for 5 minutes. Add the culture medium containing LipoHigh from the previous step to the culture medium containing circRNA, gently mix well, and let it stand at room temperature for 20 minutes. Remove the cell culture plate from the incubator, drop it into the inoculated cells, gently mix well, and place it in a 37°C, 5% CO₂ incubator for cultivation. After 4-8 hours, discard the solution and add 0.5mL of culture medium containing 2% FBS to continue culturing.
b) The results of Western blot analysis (WB) are shown in Figure 20, which shows circular RNA: MYC-circRNA, KLF4-circRNA, OCT4-P2A-SOX2-circRNA, Lin28A-P2A-Nanog-circRNA all can express corresponding proteins.

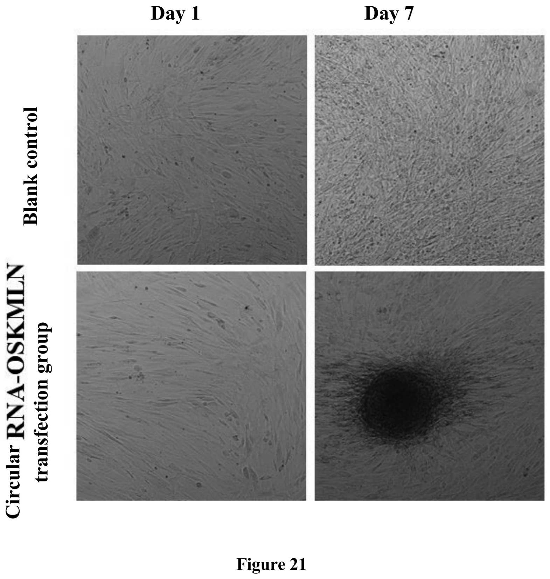

4.Wild-type OKSMLN circular RNA was transfected into fibroblasts to induce iPSCs. Functional validation experiment: human skin fibroblasts were seeded into a 12-well plate and grown to a confluence degree of 80%-90%. Using LipofectamineMessengerMAX transfection reagent (Thermo Fisher Scientific) to encapsulate the circular RNA: MYC-circRNA, KLF4-circRNA, OCT4-P2A-SOX2-circRNA, Lin28A-P2A-Nanog-circRNA, co-incubate fibroblasts at a total mRNA amount of 2µg per well for transfection. After 24 hours, terminate transfection and observe cell growth. On the 7th day after transfection, continue to observe the growth of cells and check for the appearance of clones. The results are shown in Figure 21. The results showed that wild-type OSKMLN circular RNA can successfully induce fibroblast to produce pluripotent stem cell like clones.
Circular RNA sequence

| | | |
|---|---|---|
| MYC circular RNA | MYC-circRNA | SEQ ID NO: 53 |
| KLF4 circular RNA | KLF4-circRNA | SEQ ID NO: 54 |
| Lin28A-P2A-Nanog circular RNA | LN-circRNA | SEQ ID NO: 55 |
| OCT4-P2A-SOX2 circular RNA | OS-circRNA | SEQ ID NO: 56 |
| OCT4'-P2A-SOX2-T2A-KLF4s circular RNA | O'SK-circRNA | SEQ ID NO: 57 |
| OCT4-P2A-SOX2-T2A-KLF4s circular RNA | OSK-circRNA | SEQ ID NO: 58 |
| OCT4'-P2A-KLF4s-T2A-MYC' circular RNA | O'KM'-circRNA | SEQ ID NO: 59 |
| OCT4-P2A-KLF4s-T2A-MYC circular RNA | OKM-circRNA | SEQ ID NO: 60 |
| OCT4'-P2A-KLF4s-T2A-MYC'-E2A-SOX2 circular RNA | O'KM'S-circRNA | SEQ ID NO: 61 |
| OCT4-P2A-KLF4s-T2A-MYC-E2A-SOX2 circular RNA | OKMS-circRNA | SEQ ID NO: 62 |

## Claims

1. A method of rejuvenating cells, **characterized in that**, an initiation phase reprogramming of a cell is mediated using a phase separation-deficient engineered reprogramming factor.

2. The method of claim 1, the rejuvenating is judged by an increase or a decrease in an expression level of epigenetic marker gene(s) relative to a reference value.

3. The method of claim 1, the rejuvenating is manifested by a change of the proportion of rejuvenated cells exhibiting senescence-associated β-galactosidase (SA-β-Gal) positivity, the change is a reduction relative to a reference value.

4. The method of claim 2, the rejuvenating cells is **characterized by**: a reduction in the level of epigenetic age (EpiAge) of DNA methylation and/or an increase in an expression level of a genes related to rejuvenation function; and/or an increase in an expression level of initiation phase feature genes of reprogramming; and/or a decrease in the expression level of maturation phase feature genes of reprogramming; and/or an increase in an expression level of cell identity genes, relative to a reference value.

5. The method of claim 4, wherein the genes related to rejuvenation function comprise COL1A1, COL3A1, COL4A1, FN1, and LAMA5; the feature genes that detect the initiation phase of the reprogramming comprise NANOG and SALL4; the feature genes that detect the maturation phase of the reprogramming comprise DPPA5 and LIN28A; and the identity genes that detect cell comprise THY1, P4HB, S100A4, and SERPINH1.

6. A method of reprogramming at an initiation phase of a cell, **characterized in that**, it is achieved by introducing an element comprising a phase separation-deficient engineered reprogramming factor into a cell.

7. A phase separation-deficient engineered transcription factor, **characterized in that**, it can mediate reprogramming of initiation phase of a cell.

8. The phase separation-deficient engineered transcription factor of claims 1-7, **characterized in that**, the phase separation-deficient engineered reprogramming factor, based on modifications to the phase separation function related regions of the transcription factor, causes the transcription factor to lose its ability to trigger a phase separation.

9. The engineered transcription factor of claim 8, the modification comprises an amino acid modification, a partial deletion treatment, and an ubiquitination mutation in a protein IDR region of the transcription factor.

10. The engineered transcription factor of claim 9, further, a polar charged amino acid or an acidic amino acid in the protein IDR region can be mutated; further, the polar charged amino acid or acidic amino acid can be substituted with a neutral amino acid; furthermore, the acidic amino acid such as aspartic acid (Asp) and glutamic acid (Glu) can be mutated into a neutral amino acid, such as alanine.

11. The engineered transcription factor of claim 10, positions 1-140 and/or 288-360 of a sequence shown in SEQ ID NO: 2 can be mutated, the positions includes an error range of 10-20 amino acids before and after said positions.

12. The engineered transcription factor of claim 10, at least one of positions 8, 20, 31, 108, 138, 291, 297; 26, 56, 68, 91, 96, 98, 104, 113, 125, 127, 130, 134, 135, 296, 299, 341, 343, 145, 147, 166, 188, 209, 210, 215, 224, 238, 246, 270 and 272 of the sequence shown in SEQ ID NO: 2 can be mutated.

13. The engineered transcription factor of claim 11 or 12, the sequence obtained after mutation can be shown in any of SEQ ID NOs: 4-10, or a sequence with at least 85%, 90%, or 95% or above identity that can achieve the same function.

14. The engineered transcription factor of claim 9, the modification can be a mutation mode that does not produce polyubiquitin chain modification; further, can be a substitution of lysine K with an amino acid that do not produce ubiquitin chain modification; further, it can be substituted with arginine R.

15. The engineered transcription factor of claim 14, at least one of positions 51, 52, 126, 143, 148, 157, 206, 269, 275, 289, 298, 317, 323, 326, 341, 355, 371, 389, 392, 397, 398, 412, 422, 428, and 430 of SEQ ID NO: 12 can be mutated.

16. The engineered transcription factor of claim 15, mutation of at least one of amino acids at positions 148 and 389 is further preferred; preferably amino acids at positions 20-25 are mutated; and preferably amino acids at all positions of claim 15 are mutated.

17. The engineered transcription factor of claim 16, the sequence obtained after mutation can be exemplified by variants shown in SEQ ID NOs: 14-20, or a sequence with at least 85%, 90%, or 95% or more identity that can achieve the same function.

18. The engineered transcription factor of claim 9, the partial deletion treatment involves a deletion treatment of a key regulatory region for phase separation of a protein or a deletion treatment of an amino acid that plays a critical role in formation of the phase separation.

19. The engineered transcription factor of claim 18, the partial deletion treatment is preferably a terminal deletion treatment, or a deletion treatment at the IDR region, or a deletion treatment at a non-critical region outside of the DBD; preferably, a partial deletion treatment at the charged amino acid in the IDR region or a deletion treatment at a critical amino acid for ubiquitination chain modification.

20. The engineered transcription factor of claim 19, wherein the critical amino acid for ubiquitination chain modification is lysine.

21. The engineered transcription factor of claim 19, a sequence of SOX2 shown in SEQ ID NOs: 21-22 may be subjected to a partial deletion; preferably, a partial or complete deletion treatment may be made at positions 2-40 and/or 201-317 to obtain, for example, amino acid sequences as shown in SEQ ID NOs: 41-43, or sequences with at least 85%, 90%, or 95% or above identity that can achieve the same function.

22. The engineered transcription factor of claim 19, a sequence of OCT4 shown in SEQ ID NOs: 1-2 may be subjected to a partial deletion; preferably, a partial or complete deletion treatment may be made at positions 2-140 and/or positions 288-360 at the C-terminus to obtain, for example, amino acid sequences as shown in SEQ ID NOs: 44-46, or sequences with at least 85%, 90%, or 95% or above identity that can achieve the same function.

23. The engineered transcription factor of claim 19, a sequence of c-MYC shown in SEQ ID NOs: 11-12 may be subjected to a partial deletion; preferably, a partial or complete deletion treatment may be made at positions 2-360 to obtain, for example, amino acid sequences as shown in SEQ ID NOs: 47-48, or sequences with at least 85%, 90%, or 95% or above identity that can achieve the same function.

24. A nucleic acid that can express the engineered transcription factor of claims 7-23.

25. The nucleic acid of claim 24, comprising SEQ ID NOs: 3, 13, 21, or sequences with at least 85%, 90%, or 95% or above identity that can achieve the same function.

26. An element comprising the engineered transcription factor of claims 7-25, comprising DNA, RNA, a recombinant vector, a recombinant protein, and a recombinant cell; preferably, the recombinant vector can be an adeno-associated virus vector, a poxvirus vector, a lentivirus vector, etc

27. The element of claim 25, the RNA element, further it may be a linear mRNA element, a siRNA element, a circular RNA element, a circular mRNA element, a tRNA element, etc.

28. The element of claim 25, the recombinant vector can be monocistron, bicistron or multicistron, linked by at least one linker peptide.

29. The element of claim 26, wherein the recombinant vector can be composed of multiple transcription factors linked in tandem, which can be linked with or without at least one linker peptide, further, the linker peptide can be selected from SEQ ID NOs: 25-27.

30. The engineered transcription factor of claims 7-25 or the element of claims 26-29, **characterized in that**, it is selected from at least one of OCT4, SOX2, KLF4, c-MYC, L-MYC, LIN28, NANOG, and GLIS1.

31. The engineered transcription factor or the element of claim 30, the engineered transcription factor can be sequentially linked in tandem, and the linking order can be OCT4-SOX2, LIN28-NANOG, OCT4-SOX2-KLF4, OCT4-KLF4-c-MYC, and OCT4- KLF4- c-MYC-SOX2 _{∘}

32. The engineered transcription factor or element of claim 31, the sequence in tandem can be as shown in SEQ ID NOs: 28-33, and the sequence of the recombinant vector can be as shown in SEQ ID NOs: 34-39.

33. An engineered transcription factor, at least one of the transcription factors has been modified by the transcription factors of claims 8-23.

34. A delivery system for delivering the element of claims 26-32.

35. The delivery system of claim 34, further it can be a liposome LNP, exosome delivery, a GalNAc system, VLP delivery, a protamine, a high molecular polymer, an inorganic nanoparticle, an exosome, a polymer matrix, virus transfection, etc.

36. Use of the engineered transcription factor of claims 7-25 or the element comprising the engineered transcription factor of claims 26-32, or the delivery system of claims 34-35, in cell therapy, gene therapy, immune cell proliferation, cancer therapy, treatment of a senescence-associated disease, or in the preparation of a medicament for cell therapy, gene therapy, immune cell proliferation, cancer therapy, treatment of a senescence-associated disease.

37. A pharmaceutical composition, comprising at least one of the engineered transcription factor of claims 7-25, or the element comprising the engineered transcription factor of claims 26-32, or the delivery system of claims 34-35.

38. The pharmaceutical composition of claim 37, the pharmaceutical composition can be a mixture.

39. A method of combination therapy, using the pharmaceutical composition of claims 37-38 in combination with other associated drugs.

40. A method of combination therapy, using the pharmaceutical composition of claims 37-38 in combination with a B18R protein for treatment.
